(19) Europäisches Patentamt
European Patent Office
Office européen des brevets

(11) EP 0 809 664 B1

(12) **EUROPÄISCHE PATENTSCHRIFT**

(45) Veröffentlichungstag und Bekanntmachung des Hinweises auf die Patenterteilung:
**17.05.2000 Patentblatt 2000/20**

(21) Anmeldenummer: **96903976.7**

(22) Anmeldetag: **03.02.1996**

(51) Int Cl.[7]: **C08G 63/20**, C08G 63/60, C08G 63/91

(86) Internationale Anmeldenummer:
**PCT/EP96/00457**

(87) Internationale Veröffentlichungsnummer:
**WO 96/25446 (22.08.1996 Gazette 1996/38)**

(54) **BIOLOGISCH ABBAUBARE POLYMERE, VERFAHREN ZU DEREN HERSTELLUNG SOWIE DEREN VERWENDUNG ZUR HERSTELLUNG BIOABBAUBARER FORMKÖRPER**

BIODEGRADABLE POLYMERS, PROCESS FOR PRODUCING THEM AND THEIR USE IN PREPARING BIODEGRADABLE MOULDINGS

POLYMERES BIODEGRADABLES, LEUR PROCEDE DE FABRICATION ET LEUR EMPLOI DANS LA FABRICATION DE PIECES MOULEES BIODEGRADABLES

(84) Benannte Vertragsstaaten:
**BE CH DE DK ES FR GB IT LI NL SE**

(30) Priorität: **16.02.1995 DE 19505185**

(43) Veröffentlichungstag der Anmeldung:
**03.12.1997 Patentblatt 1997/49**

(73) Patentinhaber: **BASF AKTIENGESELLSCHAFT**
**67056 Ludwigshafen (DE)**

(72) Erfinder:
- **WARZELHAN, Volker**
  **D-67273 Weisenheim (DE)**
- **PIPPER, Gunter**
  **D-67098 Bad Dürkheim (DE)**
- **SEELIGER, Ursula**
  **D-67059 Ludwigshafen (DE)**
- **BAUER, Peter**
  **D-67071 Ludwigshafen (DE)**
- **PAGGA, Udo**
  **D-67069 Ludwigshafen (DE)**
- **YAMAMOTO, Motonori**
  **D-68199 Mannheim (DE)**

(56) Entgegenhaltungen:
**EP-A- 0 028 687**
**EP-A- 0 569 143**
**WO-A-91/02015**

- **DATABASE WPI Week 9410 Derwent Publications Ltd., London, GB; AN 94-079832 XP002004708 & JP,A,06 032 357 (SHOWA HIGH POLYMER CO., LTD) , 8.Februar 1994**
- **DATABASE WPI Week 8142 Derwent Publications Ltd., London, GB; AN 81-76516D XP002004709 & JP,A,56 110 720 (TEIJIN KK) , 2.September 1981**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft biologisch abbaubare Polyester P1, erhältlich durch Reaktion einer Mischung, bestehend im wesentlichen aus

(a1) einer Mischung, bestehend im wesentlichen aus

45 bis 80 mol-% Adipinsäure oder esterbildende Derivate davon oder Mischungen davon,

20 bis 55 mol-% Terephthalsäure oder esterbildende Derivate davon oder Mischungen davon, und

0 bis 5 mol-% einer sulfonatgruppenhaltigen Verbindung,

wobei die Summe der einzelnen Molprozentangaben 100 mol-% beträgt, und

(a2) einer Dihydroxyverbindung, ausgewählt aus der Gruppe bestehend aus $C_2$-$C_6$-Alkandiolen und $C_5$-$C_{10}$-Cycloalkandiolen,

wobei man das Molverhältnis von (a1) zu (a2) im Bereich von 0,4:1 bis 1,5:1 wählt, mit der Maßgabe, daß die Polyester P1 ein Molekulargewicht ($M_n$) im Bereich von 5000 bis 50000 g/mol, eine Viskositätszahl im Bereich von 30 bis 350 g/ml (gemessen in o-Dichlorbenzol/Phenol (Gew.-Verhältnis 50/50) bei einer Konzentration von 0,5 Gew.-% Polyester P1 bei einer Temperatur von 25°C) und einen Schmelzpunkt im Bereich von 50 bis 170°C aufweisen, und mit der weiteren Maßgabe, daß man von 0,01 bis 5 mol-%, bezogen auf die Molmenge der eingesetzten Komponente (a1), eine Verbindung D mit mindestens drei zur Esterbildung befähigten Gruppen zur Herstellung der Polyester P1 einsetzt, sowie der weiteren Maßgabe, daß die Polyester P1 sowohl Hydroxyl- als auch Carboxylendgruppen besitzen, wobei man das Molverhältnis von Carboxylendgruppen zu Hydroxylendgruppen größer als eins wählt.

Des weiteren betrifft die Erfindung Polymere und biologisch abbaubare thermoplastische Formmassen gemäß Unteransprüche, Verfahren zu deren Herstellung, deren Verwendung zur Herstellung biologisch abbaubarer Formkörper sowie Klebstoffe, biologisch abbaubare Formkörper, Schäume und Blends mit Stärke, erhältlich aus den erfindungsgemäßen Polymeren bzw. Formmassen.

Polymere, die biologisch abbaubar sind, d.h. die unter Umwelteinflüssen in einer angemessenen und nachweisbaren Zeitspanne zerfallen, sind seit einiger Zeit bekannt. Der Abbau erfolgt dabei in der Regel hydrolytisch und/oder oxidativ, zum überwiegenden Teil jedoch durch die Einwirkung von Mikroorganismen wie Bakterien, Hefen, Pilzen und Algen. Y.Tokiwa und T. Suzuki (Nature, Bd. 270, S. 76-78, 1977) beschreiben den enzymatischen Abbau von aliphatischen Polyestern, beispielsweise auch Polyester auf der Basis von Bernsteinsäure und aliphatischer Diole.

In der EP-A 565,235 werden aliphatische Copolyester, enthaltend [-NH-C(O)O-]-Gruppen ("Urethan-Einheiten"), beschrieben. Die Copolyester der EP-A 565,235 werden durch Umsetzung eines Präpolyesters - erhalten durch Umsetzung von im wesentlichen Bernsteinsäure und eines aliphatischen Diols - mit einem Diisocyanat, bevorzugt Hexamethylendiisocyanat, erhalten. Die Umsezung mit dem Diisocyanat ist gemäß der EP-A 565,235 erforderlich, da durch die Polykondensation alleine nur Polymere mit solchen Molekulargewichten erhalten werden, die keine befriedigenden mechanischen Eigenschaften aufweisen. Von entscheidendem Nachteil ist die Verwendung von Bernsteinsäure oder deren Esterderivate zur Herstellung der Copolyester, weil Bernsteinsäure bzw. deren Derivate teuer und in nicht genügender Menge auf dem Markt verfügbar sind. Außerdem werden bei Verwendung von Bernsteinsäure als einziger Säurekomponente die daraus hergestellten Polyester nur extrem langsam abgebaut.

Aus der WO 92/13019 sind Copolyester auf Basis überwiegend aromatischer Dicarbonsäuren und aliphatischer Diole bekannt, wobei mindestens 85 mol-% des Polyesterdiolrestes aus einem Terephthalsäurerest bestehen. Durch Modifikationen wie den Einbau von bis zu 2,5 Mol-% Metallsalze der 5-Sulfoisophthalsäure oder kurzkettigen Etherdiol-Segmenten wie Diethylenglycol wird die Hydrophilie des Copolyesters gesteigert und die Kristallinität vermindert. Hierdurch soll gemäß der WO 92/13019 ein biologischer Abbau der Copolyester ermöglicht werden. Nachteilig an diesen Copolyestern ist jedoch, daß ein biologischer Abbau durch Mikroorganismen nicht nachgewiesen wurde, sondern lediglich das Verhalten gegenüber Hydrolyse in kochendem Wasser oder in manchen Fällen auch mit Wasser von 60°C durchgeführt wurde.

Nach Angaben von Y.Tokiwa und T.Suzuki (Nature, Bd. 270, 1977 oder J. of Appl. Polymer Science, Bd. 26, S. 441-448, 1981) ist davon auszugehen, daß Polyester, die weitgehend aus aromatischen Dicarbonsäure-Einheiten und aliphatischen Diolen aufgebaut sind, wie PET (Polyethylenterephthalat) und PBT (Polybutylenterephthalat), enzymatisch nicht abbaubar sind. Dies gilt auch für Copolyester, die Blöcke, aufgebaut aus aromatischen Dicarbonsäureeinheiten und aliphatischen Diolen, enthalten.

Witt et al. (Handout zu einem Poster auf dem International Workshop des Royal Institute of Technology, Stockholm, Schweden, vom 21. bis 23.04.94) beschreiben biologisch abbaubare Copolyester auf der Basis von 1,3-Propandiol, Terephthalsäureester und Adipin- oder Sebazinsäure. Nachteilig an diesen Copolyestern ist, daß daraus hergestellte Formkörper, insbesondere Folien, unzureichende mechanische Eigenschaften aufweisen.

Aufgabe der vorliegenden Erfindung war es daher, biologisch, d.h. durch Mikroorganismen, abbaubare Polymere bereitzustellen, die diese Nachteile nicht aufweisen. Insbesondere sollten die erfindungsgemäßen Polymere aus bekannten und preiswerten Monomerbausteinen herstellbar und wasserunlöslich sein. Des weiteren sollte es möglich sein, durch spezifische Modifikationen wie Kettenverlängerung, Einbau von hydrophilen Gruppen und verzweigend wirkenden Gruppen, maßgeschneiderte Produkte für die gewünschten erfindungsgemäßen Anwendungen zu erhalten. Dabei sollte der biologische Abbau durch Mikroorganismen nicht auf Kosten der mechanischen Eigenschaften erreicht werden, um die Zahl der Anwendungsgebiete nicht einzuschränken.

Demgemäß wurden die eingangs definierten Polymere und thermoplastischen Formmassen gefunden.

Des weiteren wurden Verfahren zu deren Herstellung, deren Verwendung zur Herstellung biologisch abbaubarer Formkörper und Klebstoffe sowie biologisch abbaubare Formkörper, Schäume, Blends mit Stärke und Klebstoffe, erhältlich aus den erfindungsgemäßen Polymeren und Formmassen, gefunden.

Die erfindungsgemäßen Polyester P1 sind charakterisiert durch ein Molekulargewicht ($M_n$) im Bereich von 5000 bis 50000, vorzugsweise von 6000 bis 45000, besonders bevorzugt von 8000 bis 35000 g/mol, eine Viskositätszahl im Bereich von 30 bis 350, vorzugsweise von 50 bis 300 g/ml (gemessen in o-Dichlorbenzol/Phenol (Gew.-Verhältnis 50/50) bei einer Konzentration von 0,5 Gew.-% Polyester P1 bei einer Temperatur von 25°C) und einen Schmelzpunkt im Bereich von 50 bis 170, vorzugsweise von 60 bis 160°C, sowie der weiteren Maßgabe, daß die Polyester P1 sowohl Hydroxyl- als auch Carboxylendgruppen besitzen, wobei man das Molverhältnis von Carboxylendgruppen zu Hydroxylendgruppen größer als eins, bevorzugt größer als zwei wählt.

Die Polyester P1 erhält man erfindungsgemäß, indem man eine Mischung, bestehend im wesentlichen aus

(a1) einer Mischung, bestehend im wesentlichen aus

45 bis 80, vorzugsweise von 45 bis 80 mol-% Adipinsäure oder esterbildende Derivate davon, insbesondere die Di-$C_1$-$C_6$-alkylester wie Dimethyl-, Diethyl-, Dipropyl-, Dibutyl-, Dipentyl- und Dihexyladipat, oder deren Mischungen, bevorzugt Adipinsäure und Dimethyladipat, oder Mischungen davon,

20 bis 55, vorzugsweise 20 bis 55 mol-%, Terephthalsäure oder esterbildende Derivate davon, insbesondere die Di-$C_1$-$C_6$-alkylester wie Dimethyl-, Diethyl-, Dipropyl-, Dibutyl-, Dipentyl- oder Dihexylterephthalat, oder deren Mischungen, bevorzugt Terephthalsäure und Dimethylterephthalat, oder Mischungen davon, und

0 bis 5, vorzugsweise von 0 bis 3, besonders bevorzugt von 0,1 bis 2 mol-% einer sulfonatgruppenhaltigen Verbindung,

wobei die Summe der einzelnen Molprozentangaben 100 mol-% beträgt, und

(a2) einer Dihydroxyverbindung, ausgewählt aus der Gruppe bestehend aus $C_2$-$C_6$-Alkandiolen und $C_5$-$C_{10}$-Cycloalkandiolen,

wobei man das Molverhältnis von (a1) zu (a2) im Bereich von 0,4:1 bis 1,5:1, vorzugsweise von 0,6:1 bis 1,1:1 wählt, zur Reaktion bringt.

Als sulfonatgruppenhaltige Verbindung setzt man üblicherweise ein Alkali- oder Erdalkalimetallsalz einer sulfonatgruppenhaltigen Dicarbonsäure oder deren esterbildende Derivate ein, bevorzugt Alkalimetallsalze der 5-Sulphoisophthalsäure oder deren Mischungen, besonders bevorzugt das Natriumsalz.

Als Dihydroxyverbindungen (a2) setzt man erfindungsgemäß eine Verbindung, ausgewählt aus der Gruppe bestehend aus $C_2$-$C_6$-Alkandiolen und $C_5$-$C_{10}$-Cycloalkandiolen, ein wie Ethylenglykol, 1,2-, 1,3-Propandiol, 1,2-, 1,4-Butandiol, 1,5-Pentandiol oder 1,6-Hexandiol, insbesondere Ethylenglykol, 1,3-Propandiol und 1,4-Butandiol, Cyclopentandiol, 1,4-Cyclohexandiol, 1,2-Cyclohexandimethanol, 1,4-Cyclohexandimethanol, sowie Mischungen daraus, ein.

Des weiteren verwendet man erfindungsgemäß 0,01 bis 5, vorzugsweise von 0,05 bis 4 mol-%, bezogen auf die Komponente (al), mindestens eine Verbindung D mit mindestens drei zur Esterbildung befähigten Gruppen.

Die Verbindungen D enthalten bevorzugt drei bis zehn funktionelle Gruppen, welche zur Ausbildung von Esterbindungen fähig sind. Besonders bevorzugte Verbindungen D haben drei bis sechs funktionelle Gruppen dieser Art im Molekül, insbesondere drei bis sechs Hydroxylgruppen und/oder Carboxylgruppen. Beispielhaft seien genannt:

Weinsäure, Citronensäure, Äpfelsäure;

Trimethylolpropan, Trimethylolethan;
Pentaerythrit;
Polyethertriole;
Glycerin;
Trimesinsäure;
Trimellitsäure, -anhydrid;
Pyromellitsäure, -dianhydrid und
Hydroxyisophthalsäure.

Beim Einsatz von Verbindungen D, die einen Siedepunkt unterhalb von 200°C aufweisen, kann bei der Herstellung der Polyester P1 ein Anteil vor der Reaktion aus dem Polykondensationsgemisch abdestillieren. Es ist daher bevorzugt, diese Verbindungen in einer frühen Verfahrensstufe wie der Umesterungs- bzw. Veresterungsstufe zuzusetzen, um diese Komplikation zu vermeiden und um die größtmögliche Regelmäßigkeit ihrer Verteilung innerhalb des Polykondensats zu erzielen.

Im Falle höher als 200°C siedender Verbindungen D können diese auch in einer späteren Verfahrensstufe eingesetzt werden.

Durch Zusatz der Verbindung D kann beispielsweise die Schmelzviskosität in gewünschter Weise verändert, die Schlagzähigkeit erhöht und die Kristallinität der erfindungsgemäßen Polymere bzw. Formmassen herabgesetzt werden.

Die Herstellung der biologisch abbaubaren Polyester P1 ist grundsätzlich bekannt (Sorensen und Campbell, "Preparative Methods of Polymer Chemistry", Interscience Publishers, Inc., New York, 1961, Seiten 111 bis 127; Encyl. of Polym. Science and Eng., Bd. 12, 2. Ed., John Wiley & Sons, 1988, S. 1 bis 75; Kunststoff-Handbuch, Band 3/1, Carl Hanser Verlag, München, 1992, S. 15 bis 23 (Herstellung von Polyestern); WO 92/13019; EP-A 568,593; EP-A 565,235; EP-A 28,687), so daß sich nähere Angaben hierüber erübrigen.

So kann man beispielsweise die Umsetzung von Dimethylestern der Komponente a1 mit der Komponente a2 ("Umesterung") bei Temperaturen im Bereich von 160 bis 230°C in der Schmelze bei Atmosphärendruck vorteilhaft unter Inertgasatmosphäre durchführen.

Vorteilhaft wird bei der Herstellung des biologisch abbaubaren Polyesters P1 ein molarer Überschuß der Komponente a2, bezogen auf die Komponente a1, verwendet, beispielsweise bis zum 2 1/2fachen, bevorzugt bis zum 1,67fachen.

Üblicherweise erfolgt die Herstellung des biologisch abbaubaren Polyesters P1 unter Zugabe von geeigneten, an sich bekannten Katalysatoren wie Metallverbindungen auf der Basis folgender Elemente wie Ti, Ge, Zn, Fe, Mn, Co, Zr, V, Ir, La, Ce, Li, und Ca, bevorzugt metallorganische Verbindungen auf der Basis dieser Metalle wie Salze organischer Säuren, Alkoxide, Acetylacetonate und ähnliches, insbesondere bevorzugt auf Basis von Zink, Zinn und Titan.

Bei Verwendung von Dicarbonsäuren oder deren Anhydride als Komponente (a1) kann deren Veresterung mit Komponente (a2) vor, gleichzeitig oder nach der Umesterung stattfinden. In einer bevorzugten Ausführungsform verwendet man das in der DE-A 23 26 026 beschriebene Verfahren zur Herstellung modifizierter Polyalkylenterephthalate.

Nach der Umsetzung der Komponenten (a1) und (a2) wird in der Regel unter vermindertem Druck oder in einem Inertgasstrom, beispielsweise aus Stickstoff, bei weiterem Erhitzen auf eine Temperatur im Bereich von 180 bis 260°C die Polykondensation bis zum gewünschten Molekulargewicht unter Berücksichtigung des Molverhältnisses der Carboxylendgruppen zu Hydroxylendgruppen, das man größer als 1, vorzugsweise größer als 2, wählt, durchgeführt.

Das gewünschte Endgruppenverhältnis kann man einstellen

- durch einen entsprechenden Überschuß der Komponente a1,
- durch eine entsprechend lange Polykondensationszeit unter gleichzeitiger Entfernung des Diols bei einem Überschuß an Komponente a2, oder
- durch Zugabe einer entsprechenden Menge an polyfunktionellen Carbonsäuren oder deren Derivate, bevorzugt Dicarbonsäureanhydride wie Bernsteinsäureanhydrid, Phthalsäureanhydrid, Pyromellitsäureanhydrid oder Trimellitsäureanhydrid, wenn Polyester P1 durch Einsatz eines Überschusses an Komponente a2 überwiegend Hydroxylendgruppen aufweist.

Um unerwünschte Abbau- und/oder Nebenreaktionen zu vermeiden, kann man in dieser Verfahrensstufe gewünschtenfalls auch Stabilisatoren zusetzen. Solche Stabilisatoren sind beispielsweise die in der EP-A 13 461, US 4,328,049 oder in B. Fortunato et al., Polymer Vol. 35, Nr. 18, S. 4006 bis 4010, 1994, Butterworth-Heinemann Ltd., beschriebenen Phosphor-Verbindungen. Diese können zum Teil auch als Deaktivatoren der oben beschriebenen Katalysatoren wirken. Beispielhaft seien genannt: Organophosphite, phosphonige Säure und phosphorige Säure. Als Verbindungen, die nur als Stabilisatoren wirken seien beispielhaft genannt: Trialkylphosphite, Triphenylphosphit, Trialkylphosphate, Triphenylphosphat und Tocopherol (Vitamin E; beispielsweise als Uvinul® 2003AO (BASF) erhältlich).

Bei der Verwendung der erfindungsgemäßen biologisch abbaubaren Copolymere, beispielsweise im Verpackungsbereich z.B. für Nahrungsmittel, ist es in der Regel wünschenswert, den Gehalt an eingesetztem Katalysator so gering als möglich zu wählen sowie keine toxischen Verbindungen einzusetzen. Im Gegensatz zu anderen Schwermetallen wie Blei, Zinn, Antimon, Cadmium, Chrom etc. sind Titan- und Zinkverbindungen in der Regel nicht toxisch ("Sax Toxic Substance Data Book", Shizuo Fujiyama, Maruzen, K.K., 360 S. (zitiert in EP-A 565,235), siehe auch Römpp Chemie Lexikon Bd. 6, Thieme Verlag, Stuttgart, New York, 9. Auflage, 1992, S. 4626 bis 4633 und 5136 bis 5143). Beispielhaft seien genannt: Dibutoxydiacetoacetoxytitan, Tetrabutylorthotitanat und Zink(II)-acetat.

Das Gewichtsverhältnis von Katalysator zu biologisch abbaubaren Polyester P1 liegt üblicherweise im Bereich von 0,01:100 bis 3:100, vorzugsweise von 0,05:100 bis 2:100, wobei bei hochaktiven Titanverbindungen auch kleinere Mengen eingesetzt werden können wie 0,0001:100.

Der Katalysator kann gleich zu Beginn der Reaktion, unmittelbar kurz vor der Abtrennung des überschüssigen Diols oder gewünschtenfalls auch in mehreren Portionen verteilt während der Herstellung der biologisch abbaubaren Polyester P1 eingesetzt werden. Gewünschtenfalls können auch verschiedene Katalysatoren oder auch Gemische davon eingesetzt werden.

Die erfindungsgemäßen biologisch abbaubaren Polyester P2 sind charakterisiert durch ein Molekulargewicht ($M_n$) im Bereich von 5000 bis 80000, vorzugsweise von 6000 bis 45000, besonders vorzugsweise von 10000 bis 40000 g/mol, eine Viskositätszahl im Bereich von 30 bis 450, vorzugsweise von 50 bis 400 g/ml (gemessen in o-Dichlorbenzol/Phenol (Gew.-Verhältnis 50/50) bei einer Konzentration von 0,5 Gew.-% Polyester P2 bei einer Temperatur von 25°C) und einen Schmelzpunkt im Bereich von 50 bis 235, vorzugsweise von 60 bis 235°C aufweisen, und sowohl Hydroxyl- als auch Carboxylendgruppen besitzen, wobei man das Molverhältnis von Carboxylendgruppen zu Hydroxylendgruppen größer als eins, vorzugsweise größer als zwei, wählt.

Die biologisch abbaubaren Polyester P2 erhält man erfindungsgemäß, indem man eine Mischung zur Reaktion bringt, bestehend im wesentlichen aus

(b1) einer Mischung, bestehend im wesentlichen aus

25 bis 80, bevorzugt von 25 bis 80, besonders bevorzugt von 30 bis 70 mol-% Adipinsäure oder esterbildende Derivate davon oder Mischungen davon,

20 bis 75, bevorzugt von 20 bis 75, besonders bevorzugt von 30 bis 70 mol-% Terephthalsäure oder esterbildende Derivate davon oder Mischungen davon, und

0 bis 5, bevorzugt von 0 bis 3, besonders bevorzugt von 0,1 bis 2 mol-% einer sulfonatgruppenhaltigen Verbindung,

wobei die Summe der einzelnen Molprozentangaben 100 mol-% beträgt,

(b2) Dihydroxyverbindung (a2),

wobei man das Molverhältnis von (b1) zu (b2) im Bereich von 0,4:1 bis 1,5:1, vorzugsweise von 0,6:1 bis 1,1:1 wählt,

(b3) von 0,01 bis 100, vorzugsweise von 0,1 bis 80 Gew.-%, bezogen auf Komponente (b1), einer Hydroxycarbonsäure B1, und

(b4) von 0 bis 5, vorzugsweise von 0 bis 4, besonders bevorzugt von 0,01 bis 3,5 mol-%, bezogen auf Komponente (b1), Verbindung D,

wobei die Hydroxycarbonsäure B1 definiert ist durch die Formeln Ia oder Ib

$$HO-[-C(O)-G-O-]_pH$$

Ia

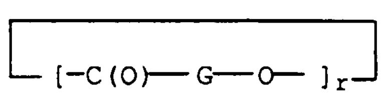

Ib

in der p eine ganze Zahl von 1 bis 1500, vorzugsweise von 1 bis 1000 und r 1, 2, 3 oder 4, vorzugsweise 1 und 2, bedeuten, und G für einen Rest steht, der ausgewählt ist aus der Gruppe bestehend aus Phenylen, $-(CH_2)_n-$, wobei n eine ganze Zahl von 1, 2, 3, 4 oder 5, vorzugsweise 1 und 5, bedeutet, -C(R)H- und $-C(R)HCH_2$, wobei R für Methyl oder Ethyl steht.

Die Herstellung der biologisch abbaubaren Polyester P2 erfolgt zweckmäßig analog zur Herstellung der Polyester P1, wobei die Zugabe der Hydroxycarbonsäure B1 sowohl zu Anfang der Umsetzung als auch nach der Veresterungs- bzw. Umesterungsstufe erfolgen kann.

In einer bevorzugten Ausführungsform setzt man als Hydroxycarbonsäure B1 ein: Glycolsäure, D-, L-, D,L-Milchsäure, 6-Hydroxyhexansäure, deren cyclische Derivate wie Glycolid (1,4-Dioxan-2,5-dion), D-, L-Dilactid (3,6-dimethyl-1,4-dioxan-2,5-dion), p-Hydroxybenzoesäure sowie deren Oligomere und Polymere wie 3-Polyhydroxybuttersäure, Polyhydroxyvaleriansäure, Polylactid (beispielsweise als EcoPLA® (Fa. Cargill) erhältlich) sowie eine Mischung aus 3-Polyhydroxybuttersäure und Polyhydroxyvaleriansäure (letzteres ist unter dem Namen Biopol® von Zeneca erhältlich), besonders bevorzugt für die Herstellung von Polyester P2 die niedermolekularen und cyclischen Derivate davon.

Die erfindungsgemäßen biologisch abbaubaren Polyester Q1 sind charakterisiert durch ein Molekulargewicht ($M_n$) im Bereich von 5000 bis 100000, vorzugsweise von 8000 bis 80000, durch eine Viskositätszahl im Bereich von 30 bis 450, vorzugsweise von 50 bis 400 g/ml (gemessen in o-Dichlorbenzol/Phenol (50/50 Gew.-%) bei einer Konzentration von 0,5 Gew.% Polyester Q1 bei einer Temperatur von 25°C), und einen Schmelzpunkt im Bereich von 50 bis 235, vorzugsweise von 60 bis 235°C, und besitzen sowohl Hydroxyl- als auch Carboxylendgruppen, wobei man das Molverhältnis von Carboxylendgruppen zu Hydroxylendgruppen größer als eins, vorzugsweise größer als 2, wählt.

Die Polyester Q1 erhält man erfindungsgemäß, indem man eine Mischung zur Reaktion bringt, bestehend im wesentlichen aus

(c1) Polyester P1 und/oder einem Polyester PWD,

(c2) 0,01 bis 50, vorzugsweise von 0,1 bis 40 Gew.-%, bezogen auf (c1), Hydroxycarbonsäure B1,

und

(c3) 0 bis 5, vorzugsweise von 0 bis 4 mol-%, bezogen auf Komponente (a1) aus der Herstellung von P1 und/oder PWD, Verbindung D.

Der biologisch abbaubare Polyester PWD ist im allgemeinen erhältlich durch Reaktion von im wesentlichen den Komponenten (a1) und (a2), wobei man das Molverhältnis von (a1) zu (a2) im Bereich von 0,4:1 bis 1,5:1, vorzugsweise von 0,6:1 bis 1,25:1 wählt, mit der Maßgabe, daß die Polyester PWD ein Molekulargewicht ($M_n$) im Bereich von 5000 bis 50000, vorzugsweise von 6000 bis 35000 g/ mol, eine Viskositätszahl im Bereich von 30 bis 350, vorzugsweise von 50 bis 300 g/ml (gemessen in o-Dichlorbenzol/Phenol (Gew.-Verhältnis 50/50) bei einer Konzentration von 0,5 Gew.-% Polyester PWD bei einer Temperatur von 25°C) und einen Schmelzpunkt im Bereich von 50 bis 170, vorzugsweise von 60 bis 160°C aufweisen, und sowohl Hydroxyl- als auch Carboxylendgruppen besitzen, wobei man das Molverhältnis von Carboxylendgruppen zu Hydroxylendgruppen größer als eins, vorzugsweise größer als 2, wählt.

Die Umsetzung der Polyester P1 und/oder PWD mit der Hydroxycarbonsäure B1 gewünschtenfalls in Gegenwart der Verbindung D erfolgt vorzugsweise in der Schmelze bei Temperaturen im Bereich von 120 bis 260°C unter Inertgasatmosphäre, gewünschtenfalls auch unter vermindertem Druck. Man kann sowohl diskontinuierlich als auch kontinuierlich, beispielsweise in Rührkesseln oder (Reaktions-)Extrudern, arbeiten.

Die Umsetzung kann gewünschtenfalls durch Zugabe an sich bekannter Umesterungskatalysatoren (siehe die weiter oben bei der Herstellung der Polyester P1 beschriebenen) beschleunigt werden.

Eine bevorzugte Ausführungsform betrifft Polyester Q1 mit Blockstrukturen gebildet aus den Komponenten P1 und B1: bei Verwendung cyclischer Derivate von B1 (Verbindungen Ib) können bei der Umsetzung mit dem biologisch abbaubaren Polyester P1 durch eine sogenannte "ringöffnende Polymerisation", ausgelöst durch die Endgruppen von P1, in an sich bekannter Weise Polyester Q1 mit Blockstrukturen erhalten werden (zur "ringöffnenden Polymerisation" siehe Encycl. of Polym. Science and Eng. Bd. 12, 2.Ed., John Wiley & Sons, 1988, S. 36 bis 41). Die Reaktion kann man gewünschtenfalls unter Zusatz üblicher Katalysatoren wie den bereits weiter oben beschriebenen Umesterungskatalysatoren durchführen, insbesondere bevorzugt ist Zinnoctanoat (siehe auch Encycl. of Polym. Science and Eng. Bd. 12, 2.Ed., John Wiley & Sons, 1988, S. 36 bis 41).

Bei Verwendung von Komponenten B1 mit höheren Molekulargewichten, beispielsweise mit einem p von größer als 10 (zehn), können durch Umsetzung mit den Polyestern P1 in Rührkesseln oder Extrudern, die gewünschten Blockstrukturen durch die Wahl der Reaktionsbedingungen wie Temperatur, Verweilzeit, Zusatz von Umesterungskatalysatoren wie den oben genannten erhalten werden. So ist aus J. of Appl. Polym. Sci., Vol. 32, S. 6191 bis 6207, John Wiley & Sons, 1986 sowie aus Makromol. Chemie, Vol. 136, S. 311 bis 313, 1970 bekannt, daß bei der Umsetzung von Polyestern in der Schmelze aus einem Blend durch Umesterungsreaktionen zunächst Blockcopolymere und dann

statistische Copolymere erhalten werden können.

Die erfindungsgemäßen biologisch abbaubaren Polyester Q2 sind charakterisiert durch ein Molekulargewicht ($M_n$) im Bereich von 6000 bis 60000, vorzugsweise von 8000 bis 50000, besonders bevorzugt von 10000 bis 40000 g/mol, durch eine Viskositätszahl im Bereich von 30 bis 340, vorzugsweise von 50 bis 300 g/ml (gemessen in o-Dichlorbenzol/Phenol (50/50 Gew.-%) bei einer Konzentration von 0,5 Gew.-% Polyester Q2 bei einer Temperatur von 25°C), und einen Schmelzpunkt im Bereich von 50 bis 170°C, vorzugsweise von 60 bis 160°C.

Die Polyester Q2 erhält man erfindungsgemäß, indem man eine Mischung zur Reaktion bringt, bestehend im wesentlichen aus

(d1) von 95 bis 99,9, vorzugsweise von 96 bis 99,8, besonders bevorzugt von 97 bis 99,65 Gew.-% Polyester P1 und/oder Polyester PWD gemäß Anspruch 3,

(d2) von 0,1 bis 5, vorzugsweise 0,2 bis 4, besonders bevorzugt von 0,35 bis 3 Gew.-% eines Bisoxazolins C1 und

(d3) von 0 bis 5, vorzugsweise von 0 bis 4 mol-%, bezogen auf Komponente (a1) aus der Herstellung von P1 und/ oder PWD, Verbindung D.

Als Bisoxazoline C1 kann man nach bisherigen Beobachtungen alle gebräuchlichen Bisoxazoline einsetzen. Entsprechende Bisoxazoline sind beispielsweise in der DE-A 39 15 874 beschrieben (unter der Bezeichnung Loxamid® im Handel erhältlich). Weitere Bisoxazoline sind in der WO 94/03523 (PCT/EP 93/01986) beschrieben.

Besonders bevorzugte Bisoxazoline C1 sind Bisoxazoline der allgemeinen Formel II

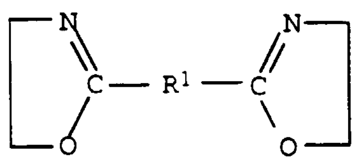

II

Die Bisoxazoline C1 der allgemeinen Formel II (Komponente d2) sind im allgemeinen erhältlich durch das Verfahren aus Angew. Chem. Int. Edit., Vol. 11 (1972), S. 287-288. Besonders bevorzugte Bisoxazoline sind solche, in denen $R^1$ eine Einfachbindung, eine $(CH_2)_q$-Alkylengruppe, mit q = 2,3 oder 4 wie Methylen, Ethan-1,2-diyl, Propan-1,3-diyl, Propan-1,2-diyl, Butan-1,4-diyl oder eine Phenylengruppe bedeutet. Als insbesonders bevorzugte Bisoxazoline seien 2,2'-Bis(2-oxazolin), Bis(2-oxazolinyl)methan, l,2-Bis(2-oxazolinyl)ethan, 1,3-Bis(2-oxazolinyl)propan, 1,4-Bis(2-oxazolinyl)butan, 1,4-Bis(2-oxazolinyl)benzol, 1,2-Bis(2-oxazolinyl)benzol und 1,3-Bis(2-oxazolinyl)benzol genannt.

Die Umsetzung der Polyester P1 und/oder PWD mit dem Bisoxazolin C1 erfolgt vorzugsweise in der Schmelze (siehe auch: J. Appl. Polym. Science, Vol. 33, S. 3069-3079 (1987)), wobei darauf zu achten ist, daß möglichst keine Nebenreaktionen auftreten, die zu einer Vernetzung oder Gelbildung führen können. In einer besonderen Ausführungsform führt man die Reaktion üblicherweise bei Temperaturen im Bereich von 120 bis 260°C, vorzugsweise von 130 bis 240°C, besonders bevorzugt 140 bis 220°C durch, wobei die Zugabe des Bisoxazolins vorteilhaft in mehreren Portionen oder kontinuierlich erfolgt.

Gewünschtenfalls kann man die Umsetzung der Polyesters P1 und/oder der PWD mit dem Bisoxazolin C1 auch in Gegenwart von gängigen inerten Lösemitteln wie Toluol, Methylethylketon oder Dimethylformamid ("DMF") oder deren Mischungen durchführen, wobei man die Reaktionstemperatur in der Regel im Bereich von 80 bis 200, vorzugsweise von 90 bis 150°C wählt.

Die Umsetzung mit dem Bisoxazolin C1 kann diskontinuierlich oder kontinuierlich beispielsweise in Rührkesseln, Reaktionsextrudern oder über Mischköpfe durchgeführt werden.

Obwohl das theoretische Optimum für die Reaktion von P1 und/oder PWD mit Bisoxazolinen C1 bei einem Molverhältnis der Oxazolin-Funktion zu P1- (bzw. PWD-)Carboxylendgruppe (besonders bevorzugt sind Polyester P1 und/oder PWD mit überwiegend Carboxyl-Endgruppen) von 1:1 liegt, kann die Umsetzung ohne technische Probleme auch bei Molverhältnissen von 1:3 bis 1,5:1 durchgeführt werden. Bei den erfindungsgemäßen Molverhältnissen von >1:1, vorzugsweise >2:1, kann gewünschtenfalls während der Umsetzung oder auch nach der Umsetzung die Zugabe einer Dicarbonsäure, bevorzugt ausgewählt aus der Gruppe aus Adipinsäure, Bernsteinsäure, Terephthalsäure und Isophthalsäure, erfolgen.

Die erfindungsgemäßen biologisch abbaubaren Polymere T1 sind charakterisiert durch ein Molekulargewicht ($M_n$) im Bereich von 10000 bis 100000, vorzugsweise von 11000 bis 80000, vorzugsweise von 11000 bis 50000 g/mol,

mit einer Viskositätszahl im Bereich von 30 bis 450, vorzugsweise von 50 bis 400 g/ml (gemessen in o-Dichlorbenzol/Phenol (Gew.-Verhältnis 50/50) bei einer Konzentration von 0,5 Gew.-% Polymer T1 bei einer Temperatur von 25°C) und einem Schmelzpunkt im Bereich von 50 bis 235, vorzugsweise von 60 bis 235°C.

Die biologisch abbaubaren Polymere T1 erhält man erfindungsgemäß, indem man einen Polyester Q1 gemäß Anspruch 3 mit

(e1) 0,1 bis 5, vorzugsweise von 0,2 bis 4, besonders bevorzugt von 0,3 bis 2,5 Gew.-%, bezogen auf den Polyester Q1, Bisoxazolin C1 sowie mit

(e2) 0 bis 5, vorzugsweise von 0 bis 4 mol-%, bezogen auf Komponente (a1) aus der Herstellung von P1 und/oder PWD sowie Polyester Q1, Verbindung D zur Reaktion bringt.

Auf diese Weise wird üblicherweise eine Kettenverlängerung erreicht, wobei die erhaltenen Polymerketten vorzugsweise eine Blockstruktur aufweisen.

Die Umsetzung erfolgt in der Regel analog zur Herstellung der Polyester Q2.

Die erfindungsgemäßen biologisch abbaubaren Polymere T2 sind charakterisiert durch ein Molekulargewicht ($M_n$) im Bereich von 10000 bis 100000, vorzugsweise von 11000 bis 80000, besonders bevorzugt von 11000 bis 50000 g/mol, mit einer Viskositätszahl im Bereich von 30 bis 450, vorzugsweise von 50 bis 400 g/ml (gemessen in o-Dichlorbenzol/Phenol (Gew.-Verhältnis 50/50) bei einer Konzentration von 0,5 Gew.-% Polymer T2 bei einer Temperatur von 25 °C) und einem Schmelzpunkt im Bereich von 50 bis 235, vorzugsweise von 60 bis 235°C.

Die biologisch abbaubaren Polymere T2 erhält man erfindungsgemäß durch Umsetzung des Polyesters Q2 mit

(f1) 0,01 bis 50, vorzugsweise von 0,1 bis 40 Gew.-%, bezogen auf den Polyester Q2, der Hydroxycarbonsäure B1 sowie mit

(f2) 0 bis 5, vorzugsweise von 0 bis 4 mol-%, bezogen auf Komponente (a1) aus der Herstellung von P1 und/oder PWD sowie des Polyesters Q2, Verbindung D,

wobei man zweckmäßig analog zur Umsetzung von Polyester P1 mit Hydroxycarbonsäure B1 zu Polyester Q1 verfährt.

Die erfindungsgemäßen biologisch abbaubaren Polymere T3 sind charakterisiert durch ein Molekulargewicht ($M_n$) im Bereich von 10000 bis 100000, vorzugsweise von 11000 bis 80000 g/mol, eine Viskositätszahl im Bereich von 30 bis 450, vorzugsweise von 50 bis 400 g/ml (gemessen in o-Dichlorbenzol/Phenol (Gew.-Verhältnis 50/50) bei einer Konzentration von 0,5 Gew.-% Polymer T3 bei einer Temperatur von 25°C) und einem Schmelzpunkt im Bereich von 50 bis 235, vorzugsweise von 60 bis 235°C.

Die biologisch abbaubaren Polymere T3 erhält man erfindungsgemäß, indem man (g1) Polyester P2, oder (g2) einer Mischung bestehend im wesentlichen aus Polyester P1 und 0,01 bis 50, vorzugsweise von 0,1 bis 40 Gew.-%, bezogen auf den Polyester P1, Hydroxycarbonsäure B1, oder (g3) einer Mischung, bestehend im wesentlichen aus Polyestern P1, die eine unterschiedliche Zusammensetzung voneinander aufweisen, mit

0,1 bis 5, vorzugsweise von 0,2 bis 4, besonders bevorzugt von 0,3 bis 2,5 Gew.-%, bezogen auf die Menge der eingesetzten Polyester, Bisoxazolin C1 sowie

mit 0 bis 5, vorzugsweise von 0 bis 4 mol-%, bezogen auf die jeweiligen Molmengen an Komponente (a1), die zur Herstellung der eingesetzten Polyester (g1) bis (g3) eingesetzt wurden, Verbindung D, zur Reaktion bringt, wobei man die Umsetzungen zweckmäßig analog zur Herstellung der Polyester Q2 aus den Polyestern P1 und/oder PWD und den Bisoxazolinen C1 vornimmt.

In einer bevorzugten Ausführungsform setzt man Polyester P2 ein, deren wiederkehrende Einheiten statistisch im Molekül verteilt sind.

Man kann jedoch auch Polyester P2 einsetzen, deren Polymerketten Blockstrukturen aufweisen. Solche Polyester P2 sind im allgemeinen zugänglich durch entsprechende Wahl, insbesondere des Molekulargewichts, der Hydroxycarbonsäure B1. So erfolgt nach bisherigen Beobachtungen im allgemeinen bei Verwendung einer hochmolekularen Hydroxycarbonsäure B1, insbesondere mit einem p von größer als 10, nur eine unvollständige Umesterung, beispielsweise auch in Gegenwart der oben beschriebenen Deaktivatoren (siehe J.of Appl. Polym. Sc. Vol. 32, S. 6191 bis 6207, John Wiley & Sons, 1986, und Makrom. Chemie, Vol. 136, S. 311 bis 313, 1970). Gewünschtenfalls kann man die Umsetzung auch in Lösung mit den bei der Herstellung der Polymeren T1 aus den Polyestern Q1 und den Bisoxazolin C1 genannten Lösungsmitteln durchführen.

Die biologisch abbaubaren thermoplastischen Formmassen T4 erhält man erfindungsgemäß, indem man in an sich bekannter Weise, bevorzugt unter Zusatz üblicher Additive wie Stabilisatoren, Verarbeitungshilfsmitteln, Füllstoffen etc. (siehe J. of Appl. Polym. Sc., Vol. 32, S. 6191 bis 6207, John Wiley & Sons, 1986; WO 92/0441; EP 515,203; Kunststoff-Handbuch, Bd. 3/1, Carl Hanser Verlag München, 1992, S. 24 bis 28)

(h1) 99,5 bis 0,5 Gew.-% Polyester P1 gemäß Anspruch 1 oder Polyester Q2 gemäß Anspruch 4 oder Polyester PWD gemäß Anspruch 3 mit

(h2) 0,5 bis 99,5 Gew.-% Hydroxycarbonsäure B1 mischt.

In einer bevorzugten Ausführungsform setzt man hochmolekulare Hydroxycarbonsäuren B1 wie Polycaprolacton oder Polylactid oder Polyglykolid oder Polyhydroxyalkanoate wie 3-Polyhydroxybuttersäure mit einem Molekulargewicht ($M_n$) im Bereich von 10000 bis 150000, vorzugsweise von 10000 bis 100000 g/mol, oder eine Mischung aus 3-Polyhydroxybuttersäure und Polyhydroxyvaleriansäure ein.

Aus WO 92/0441 und EP-A 515,203 ist es bekannt, daß hochmolekulares Polylactid ohne Zusätze von Weichmachern für die meisten Anwendungen zu spröde ist. In einer bevorzugten Ausführungsform kann man ein Blend ausgehend von 0,5 bis 20, vorzugsweise von 0,5 bis 10 Gew.-% Polyester P1 gemäß Anspruch 1 oder Polyester Q2 gemäß Anspruch 4 oder Polyester PWD gemäß Anspruch 3 und 99,5 bis 80, vorzugsweise von 99,5 bis 90 Gew.-% Polylactid herstellen, das eine deutliche Verbesserung der mechanischen Eigenschaften, beispielsweise eine Erhöhung der Schlagzähigkeit, gegenüber reinem Polylactid aufweist.

Eine weitere bevorzugte Ausführungsform betrifft ein Blend, erhältlich durch Mischen von 99,5 bis 40, vorzugsweise von 99,5 bis 60 Gew.-% Polyester P1 gemäß Anspruch 1 oder Polyester Q2 gemäß Anspruch 4 oder Polyester PWD gemäß Anspruch 3 und von 0,5 bis 60, vorzugsweise von 0,5 bis 40 Gew.-% einer hochmolekularen Hydroxycarbonsäure B1, besonders bevorzugt Polylactid, Polyglycolid, 3-Polyhydroxybuttersäure und Polycaprolacton. Solche Blends können vollständig biologisch abgebaut werden und weisen nach den bisherigen Beobachtungen sehr gute mechanische Eigenschaften auf.

Nach bisherigen Beobachtungen erhält man die erfindungsgemäßen thermoplastischen Formmassen T4 bevorzugt dadurch, daß man kurze Mischzeiten einhält, beispielsweise bei einer Durchführung des Mischens in einem Extruder. Durch Wahl der Mischparameter, insbesondere der Mischzeit und gewünschtenfalls der Verwendung von Deaktivatoren, sind auch Formmassen zugänglich, die überwiegend Blendstrukturen aufweisen, d.h., daß der Mischvorgang so gesteuert werden kann, daß zumindest teilweise auch Umesterungsreaktionen stattfinden können.

In einer weiteren bevorzugten Ausführungsform kann man 0 bis 50, vorzugsweise 0 bis 30 Mol-% der Adipinsäure, oder ihrer esterbildende Derivate oder deren Mischungen, durch mindestens eine andere aliphatische $C_4$-$C_{10}$- oder cycloaliphatische $C_5$-$C_{10}$-Dicarbonsäure oder Dimerfettsäure wie Bernsteinsäure, Glutarsäure, Pimelinsäure, Korksäure, Azelainsäure oder Sebazinsäure oder ein Esterderivat wie deren Di-$C_1$-$C_6$-alkylester oder deren Anhydride wie Bernsteinsäureanhydrid, oder deren Mischungen, ersetzen, bevorzugt Bernsteinsäure, Bernsteinsäureanhydrid, Sebacinsäure, Dimerfettsäure und Di-$C_1$-$C_6$-alkylester wie Dimethyl-, Diethyl-, Di-n-propyl-, Diisobutyl-, Di-n-pentyl-, Dineopentyl-, Di-n-hexylester davon, insbesondere Dimethylbernsteinsäure.

Eine besonders bevorzugte Ausführungsform betrifft den Einsatz als Komponente (a1) die in der EP-A 7445 beschriebene Mischung aus Bernsteinsäure, Adipinsäure und Glutarsäure sowie deren $C_1$-$C_6$-Alkylester, insbesondere der Dimethylester und Diisobutylester.

In einer weiteren bevorzugten Ausführungsform kann man 0 bis 50, vorzugsweise 0 bis 40 Mol-% der Terephthalsäure oder ihrer esterbildende Derivate, oder deren Mischungen durch mindestens eine andere aromatische Dicarbonsäure wie Isophthalsäure, Phthalsäure oder 2,6-Naphthalindicarbonsäure, bevorzugt Isophthalsäure, oder ein Esterderivat wie einen Di-$C_1$-$C_6$-alkylester, insbesondere den Dimethylester, oder deren Mischungen, ersetzen.

Allgemein sei angemerkt, daß man die unterschiedlichen erfindungsgemäßen Polymere wie üblich aufarbeiten kann, indem man die Polymere isoliert, oder, insbesondere, wenn man die Polyester P1, P2, Q1 und Q2 weiter umsetzen möchte, indem man die Polymere nicht isoliert, sondern gleich weiterverarbeitet.

Die erfindungsgemäßen Polymere kann man durch Walzen, Streichen, Spritzen oder Gießen auf Beschichtungsunterlagen aufbringen. Bevorzugte Beschichtungsunterlagen sind solche, die kompostierbar sind oder verrotten wie Formkörper aus Papier, Cellulose oder Stärke.

Die erfindungsgemäßen Polymere können außerdem zur Herstellung von Formkörpern verwendet werden, die kompostierbar sind. Als Formkörper seien beispielhaft genannt: Wegwerfgegenstände wie Geschirr, Besteck, Müllsäcke, Folien für die Landwirtschaft zur Ernteverfrühung, Verpackungsfolien und Gefäße für die Anzucht von Pflanzen.

Des weiteren kann man die erfindungsgemäßen Polymere in an sich bekannter Weise zu Fäden verspinnen. Die Fäden kann man gewünschtenfalls nach üblichen Methoden verstrecken, streckzwirnen, streckspulen, streckschären, streckschlichten und strecktexturieren. Die Verstreckung zu sogenanntem Glattgarn kann dabei in ein und demselben Arbeitsgang (fully drawn yarn oder fully oriented yarn), oder in einem getrennten Arbeitsgang erfolgen. Das

Streckschären, Streckschlichten und die Strecktexturierung führt man im allgemeinen in einem vom Spinnen getrennten Arbeitsgang durch. Die Fäden kann man in an sich bekannter Weise zu Fasern weiterverarbeiten. Aus den Fasern sind dann Flächengebilde durch Weben, Wirken oder Stricken zugänglich.

Die oben beschriebenen Formkörper, Beschichtungsmittel und Fäden etc. können gewünschtenfalls auch Füllstoffe enthalten, die man während des Polymerisationsvorganges in irgendeiner Stufe oder nachträglich, beispielsweise in eine Schmelze der erfindungsgemäßen Polymere einarbeiten kann.

Bezogen auf die erfindungsgemäßen Polymere kann von
0 bis 80 Gew.-% Füllstoffe zusetzen. Geeignete Füllstoffe sind beispielsweise Ruß, Stärke, Ligninpulver, Cellulosefasern, Naturfasern wie Sisal und Hanf, Eisenoxide, Tonmineralien, Erze, Calciumcarbonat, Calciumsulfat, Bariumsulfat und Titandioxid. Die Füllstoffe können zum Teil auch Stabilisatoren wie Tocopherol (Vitamin E), organische Phosphorverbindungen, Mono-, Di- und Polyphenole, Hydrochinone, Diarylamine, Thioether, UV-Stabilisatoren, Nukleierungsmittel wie Talkum sowie Gleit- und Formtrennmittel auf Basis von Kohlenwasserstoffen, Fettalkoholen, höheren Carbonsäuren, Metallsalzen höherer Carbonsäuren wie Calcium- und Zinkstearat, und Montanwachsen enthalten. Solche Stabilisatoren etc. sind in Kunststoff-Handbuch, Bd. 3/1, Carl Hanser Verlag, München, 1992, S. 24 bis 28 ausführlich beschrieben.

Die erfindungsgemäßen Polymere können außerdem durch den Zusatz von organischen oder anorganischen Farbstoffen beliebig eingefärbt werden. Die Farbstoffe können im weitesten Sinne auch als Füllstoff angesehen werden.

Ein besonderes Anwendungsgebiet der erfindungsgemäßen Polymere betrifft die Verwendung als kompostierbare Folie oder einer kompostierbaren Beschichtung als Außenlage von Windeln. Die Außenlage der Windeln verhindert wirksam den Durchtritt von Flüssigkeiten, die im Innern der Windel vom Fluff und Superabsorbern, bevorzugt von bioabbaubaren Superabsorbern, beispielsweise auf Basis von vernetzter Polyacrylsäure oder vernetztem Polyacrylamid, absorbiert werden. Als Innenlage der Windel kann man ein Faservlies aus einem Cellulosematerial verwenden. Die Außenlage der beschriebenen Windeln ist biologisch abbaubar und damit kompostierbar. Sie zerfällt beim Kompostieren, so daß die gesamte Windel verrottet, während mit einer Außenlage aus beispielsweise Polyethylen versehene Windeln nicht ohne vorherige Zerkleinerung oder aufwendige Abtrennung der Polyethylenfolie kompostiert werden können.

Eine weitere bevorzugte Verwendung der erfindungsgemäßen Polymere und Formmassen betrifft die Herstellung von Klebstoffen in an sich bekannter Weise (siehe beispielsweise Encycl. of Polym. Sc. and Eng. Vol.1, "Adhesive Compositions", S. 547 bis 577). Analog zur Lehre der EP-A 21042 kann man die erfindungsgemäßen Polymere und Formmassen auch mit geeigneten klebrigmachenden thermoplastischen Harzen, bevorzugt Naturharzen, nach dort beschriebenen Methoden verarbeiten. Analog zur Lehre der DE-A 4,234,305 kann man die erfindungsgemäßen Polymere und Formmassen auch zu lösungsmittelfreien Klebstoffsystemen wie Hot-melt-Folien weiterverarbeiten.

Ein weiteres bevorzugtes Anwendungsgebiet betrifft die Herstellung vollständig abbaubarer Blends mit Stärkemischungen (bevorzugt mit thermoplastischer Stärke wie in der WO 90/05161 beschrieben) analog zu dem in der DE-A 42 37 535 beschriebenen Verfahren. Die erfindungsgemäßen Polymere und thermoplastischen Formmassen lassen sich nach bisherigen Beobachtungen auf Grund ihrer hydrophoben Natur, ihren mechanischen Eigenschaften, ihrer vollständigen Bioabbaubarkeit, ihrer guten Verträglichkeit mit thermoplastischer Stärke und nicht zuletzt wegen ihrer günstigen Rohstoffbasis vorteilhaft als synthetische Blendkomponente einsetzen.

Weitere Anwendungsgebiete betreffen beispielsweise die Verwendung der erfindungsgemäßen Polymere in landwirtschaftlichem Mulch, Verpackungsmaterial für Saatgut und Nährstoffe, Substrat in Klebefolien, Babyhöschen, Taschen, Bettücher, Flaschen, Kartons, Staubbeutel, Etiketten, Kissenbezüge, Schutzkleidung, Hygieneartikel, Taschentücher, Spielzeug und Wischer.

Eine weitere Verwendung der erfindungsgemäßen Polymere und Formmassen betrifft die Herstellung von Schäumen, wobei man im allgemeinen nach an sich bekannten Methoden vorgeht (siehe EP-A 372,846; Handbook of Polymeric foams and Foam Technology, Hanser Publisher, München, 1991, S. 375 bis 408). Üblicherweise wird dabei das erfindungsgemäße Polymere bzw. Formmasse zunächst aufgeschmolzen, gewünschtenfalls unter Zugabe von bis zu 5 Gew.-% Verbindung D, bevorzugt Pyromellitsäuredianhydrid und Trimellitsäureanhydrid, dann mit einem Treibmittel versetzt und die so erhaltene Mischung durch Extrusion vermindertem Druck ausgesetzt, wobei die Schäumung entsteht.

Die Vorteile der erfindungsgemäßen Polymere gegenüber bekannten bioabbaubaren Polymere liegen in einer günstigen Rohstoffbasis mit gut verfügbaren Ausgangsstoffen wie Adipinsäure, Terephthalsäure und gängigen Diolen, in interessanten mechanischen Eigenschaften durch Kombination von "harten" (durch die aromatischen Dicarbonsäuren wie beispielsweise Terephthalsäure) und "weichen" (durch die aliphatischen Dicarbonsäuren, wie beispielsweise Adipinsäure) Segmenten in der Polymerkette und der Variation der Anwendungen durch einfache Modifizierungen, in einem guten Abbau-> verhalten durch Mikroorganismen, besonders im Kompost und im Boden, und in einer gewissen Resistenz gegenüber Mikroorganismen in wäßrigen Systemen bei Raumtemperatur, was für viele Anwendungsbereiche besonders vorteilhaft ist. Durch den statistischen Einbau der aromatischen Dicarbonsäuren der Komponenten (a1) in verschiedenen Polymeren wird der biologische Angriff ermöglicht und damit die gewünschte biologische Abbaubarkeit erreicht.

Besonders vorteilhaft an den erfindungsgemäßen Polymere ist, daß durch maßgeschneiderte Rezepturen sowohl biologisches Abbauverhalten und mechanische Eigenschaften für den jeweiligen Anwendungszweck optimiert werden können.

Des weiteren können je nach Herstellverfahren vorteilhaft Polymere mit überwiegend statistisch verteilten Monomerbausteinen, Polymere mit überwiegend Blockstrukturen sowie Polymere mit überwiegend Blendstruktur oder Blends erhalten werden.

Beispiele

Enzym-Test

Die Polymere wurden in einer Mühle mit flüssigem Stickstoff oder Trockeneis gekühlt und fein gemahlen (je größer die Oberfläche des Mahlguts, desto schneller der enzymatische Abbau). Zur eigentlichen Durchführung des Enzym-Tests wurden 30 mg fein gemahlenes Polymerpulver und 2 ml einer 20 mmol wäßrigen $K_2HPO_4$/ $KH_2PO_4$-Pufferlösung (pH-Wert: 7,0) in ein Eppendorfreagenzgefäß (2 ml) gegeben und 3 h bei 37°C auf einem Schwenker equilibriert Anschließend wurden 100 units Lipase aus entweder Rhizopus arrhizus, Rhizopus delemar oder Pseudomonas pl. zugesetzt und 16 h bei 37°C unter Rühren (250 rpm) auf dem Schwenker inkubiert. Danach wurde die Reaktionsmischung durch eine Millipore®-Membran (0,45 µm) filtriert und der DOC (dissolved organic carbon) des Filtrats gemessen. Analog dazu wurden je eine DOC-Messung nur mit Puffer und Enzym (als Enzymkontrolle) und eine nur mit Puffer und Probe (als Blindwert) durchgeführt.

Die ermittelten ΔDOC-Werte (DOC(Probe + Enzym)-DOC(Enzymkontrolle)-DOC(Blindwert)) können als Maß für die enzymatische Abbaubarkeit der Proben angesehen werden. Sie sind jeweils im Vergleich zu einer Messung mit Pulver von Polycaprolacton® Tone P 787 (Union Carbide) dargestellt. Bei der Bewertung ist darauf zu achten, daß es sich nicht um absolut quantifizierbare Daten handelt. Auf den Zusammenhang zwischen Oberfläche des Mahlguts und Schnelligkeit des enzymatischen Abbaus wurde weiter oben bereits hingewiesen. Des weiteren können auch die Enzymaktivitäten schwanken.

Die Molekulargewichte wurden mittels Gelpermeationschromatographie (GPC) gemessen:

| | |
|---|---|
| stationäre Phase: | 5 MIXED B-Polystyrolgelsäulen (7,5x300 mm, PL-gel 10 µ) der Fa. Polymer Laboratories; Temperierung: 35°C. |
| mobile Phase: | Tetrahydrofuran (Fluß: 1,2 ml/min) |
| Eichung: | Molgewicht 500-10000000 g/mol mit PS-Eichkit der Fa. Polymer Laboratories. |

Im Oligomerbereich Ethylbenzol/1,3-Diphenylbutan/1,3,5-Triphenylhexan/1,3,5,7-Tetraphenyloktan/1,3,5,7,9-Pentaphenyldekan

| | |
|---|---|
| Detektion: | RI (Brechungsindex) Waters 410 |
| | UV (bei 254 nm) Spectra Physics 100 |

Die Bestimmungen der Hydroxyl-Zahl (OH-Zahl) und Säure-Zahl (SZ) erfolgten nach folgenden Methoden:

(a) Bestimmung der scheinbaren Hydroxyl-Zahl

Zu ca. 1 bis 2 g exakt eingewogener Prüfsubstanz wurden 10 ml Toluol und 9,8 ml Acetylierungsreagenz (s. u.) gegeben und 1 h bei 95°C unter Rühren erhitzt. Danach wurden 5 ml dest. Wasser zugeführt. Nach Abkühlen auf Raumtemperatur wurden 50 ml Tetrahydrofuran (THF) zugesetzt und mit ethanolischer KOH-Maßlösung gegen Wendepunkt potentiographisch titriert.

Der Versuch wurde ohne Prüfsubstanz wiederholt (Blindprobe).

Die scheinbare OH-Zahl wurde dann aufgrund folgender Formel ermittelt:

scheinb. OH-Zahl $c \cdot t \cdot 56{,}1 \cdot (V2-V1)/m$ (in mg KOH/g)

wobei c = Stoffmengenkonzentration der ethanol. KOH-Maßlösung in mol/l,

t = Titer der ethanol. KOH-Maßlösung

m = Einwaage in mg der Prüfsubstanz

V1 = Verbrauch der Maßlösung mit Prüfsubstanz in ml

V2 = Verbrauch der Maßlösung ohne Prügsubstanz in ml bedeuten.

Verwendete Reagenzien:

ethanol. KOH-Maßlösung, c - 0,5 mol/l, Titer 0,9933 (Merck, Art.Nr. 1.09114)

Essigsäureanhydrid p.A. (Merck, Art.Nr. 42)

Pyridin p.A. (Riedel de Haen, Art.-Nr 33638)
Essigsäure p.A. (Merck, Art.Nr. 1.00063)
Acetylierungsreagenz: 810 ml Pyridin, 100 ml
Essigsäureanhydrid und 9 ml
Essigsäure
Wasser, deionisiert
THF und Toluol
(b) Bestimmung der Säurezahl (SZ)

Ca. 1 bis 1,5 g Prüfsubstanz wurden exakt eingewogen und mit 10 ml Toluol und 10 ml Pyridin versetzt und anschließend auf 95°C erhitzt. Nach dem Lösen wurde auf Raumtemperatur abgekühlt, 5 ml Wasser und 50 ml THF zugegeben und mit 0,1 N ethanol. KOHMaßlösung titriert.

Die Bestimmung wurde ohne Prüfsubstanz wiederholt (Blindprobe)

Die Säure-Zahl wurde dann aufgrund folgender Formel ermittelt:

$$SZ = c \cdot t \cdot 56{,}1 \cdot (V1-V2)/m \text{ (in mg KOH/g)}$$

wobei

c = Stoffmengenkonzentration der ethanol. KOH-Maßlösung in mol/l,

t = Titer der ethanol. KOH-Maßlösung

m = Einwaage in mg der Prüfsubstanz

V1 = Verbrauch der Maßlösung mit Prüfsubstanz in ml

V2 = Verbrauch der Maßlösung ohne Prüfsubstanz in ml bedeuten.

Verwendete Reagenzien:
ethanol. KOH-Maßlösung, c - 0,1 mol/l, Titer - 0,9913 (Merck, Art.Nr. 9115)
Pyridin p.A. (Riedel de Haen, Art.Nr. 33638)
Wasser, deionisiert
THF und Toluol
(c) Bestimmung der OH-Zahl

Die OH-Zahl ergibt sich aus der Summe der scheinbaren OH-Zahl und der SZ:
OH-Zahl = scheinb. OH-Zahl + SZ

Verwendete Abkürzungen:

DOC: dissolved organic carbon
DMT: Dimethylterephthalat
PCL: Polycaprolacton® Tone P 787 (Union Carbide)
PMDA: Pyromellitsäuredianhydrid
SZ: Säurezahl
TBOT: Tetrabutylorthotitanat
VZ: Viskositätszahl (gemessen in o-Dichlorbenzol/Phenol (Gew.-Verhältnis 50/50) bei einer Konzentration von 0,5 Gew.-% Polymer bei einer Temperatur von 25°C
$T_m$: "Schmelztemperatur" - Temperatur, bei der ein maximaler endothermer Wärmefluß auftritt (Extremum der DSC-Kurven)
$T_g$: Glasübergangstemperatur (midpoint der DSC-Kurven)

Die DSC-Messungen wurden mit einem DSC-Gerät 912+Thermal Analyzer 990 der Fa. DuPont durchgeführt. Die Temperatur- und Enthalpiekalibrierung erfolgte in üblicher Weise. Die Probeneinwaage betrug typischerweise 13 mg. Heiz- und Kühlraten betrugen - außer wenn anders vermerkt - 20 K/min. Die Proben wurden unter folgenden Bedingungen vermessen: 1. Aufheizender Lauf an Proben im Anlieferungszustand, 2. Schnelles Abkühlen aus der Schmelze, 3. Aufheizender Lauf an aus der Schmelze abgekühlten Proben (Proben aus 2). Die jeweils zweiten DSC-Läufe dienten dazu, nach Einprägen einer einheitlichen thermischen Vorgeschichte, einen Vergleich zwischen den

verschiedenen Proben zu ermöglichen.

Beispiel 1 - Herstellung eines Polyesters P1

(a) 4672 kg 1,4 Butandiol, 7000 kg Adipinsäure und 50 g Zinndioctoat wurden in einer Stickstoffatmosphäre bei einer Temperatur im Bereich von 230 bis 240°C zur Reaktion gebracht. Nach Abdestillieren der Hauptmenge des bei der Umsetzung gebildeten Wassers, wurden 10 g TBOT zur Reaktionsmischung gegeben. Nachdem die Säurezahl unter den Wert 1 gesunken war, wurde unter vermindertem Druck überschüssiges 1,4-Butandiol solange abdestilliert, bis eine OH-Zahl von 56 erreicht war.

(b) 1,81 kg des Polyesters aus Bsp. la, 1,17 kg DMT, 1,7 kg 1,4-Butandiol und 4,7 g TBOT sowie 6,6 g PMDA wurden in einen Dreihalskolben gegeben und unter Stickstoffatmosphäre mit langsamen Rühren auf 180°C erhitzt. Dabei wurde das während der Umesterungsreaktion gebildete Methanol abdestilliert. Innerhalb von 2 h wurde unter Erhöhung der Rührgeschwindigkeit auf 230°C erhitzt und nach einer weiteren Stunde noch 2 g 50 gew.-%ige wäßrige phosphorige Säure zugegeben. Innerhalb von 1 h wurde der Druck auf 5 mbar abgesenkt und bei 240°C noch 2,5 h < 2 mbar gehalten, wobei das im Überschuß eingesetzte 1,4-Butandiol abdestillierte.

OH-Zahl: 1 mg KOH/g
SZ: 8,8 mg KOH/g
VZ: 98,2 g/ml
$T_m$: 93°C (DSC, Anlieferungszustand), $T_g$: -39°C

Beispiel 2 - Herstellung eines Polyesters Q2

Zu 300 g des Copolyesters aus Beispiel la wurden unter Rühren und unter Stickstoffatmosphäre bei 200°C tropfenweise innerhalb von 30 min 26,25 g des Bisoxalins Bis(2-ricinol-2-oxazolin)-tetramethylxyloldiurethan (Loxamid® VEP 8523 der Fa. Henkel, ein Bisoxazolin aus Ricinololoxazolin und 4,4'-Diphenylmethandiisocyanat, herstellbar gemäß DE-A 39 15 874) zugegeben, wobei die Schmelzviskosität zunahm und sich das Produkt bräunlich färbte.

OH-Zahl: 2 mg KOH/g
SZ: 1,9 mg KOH/g
$T_m$: 97°C, $T_g$: -34°C (DSC, von 190°C schnell abgekühlt)
Enzym-Test mit Rhizopus arrhizus: ΔDOC: 357 mg/l; zum Vergleich mit PCL: ΔDOC: 2588 mg/l.

Beispiel 3 - Herstellung eines weiteren Polyesters Q2

Zu 300 g des Copolyesters aus Beispiel la wurden unter Rühren und unter Stickstoffatmosphäre bei 200°C tropfenweise innerhalb von 10 min 6,9 g des Bisoxalins 1,4-Bis(2-oxazolin)butan zugegeben, wobei die Schmelzviskosität zunahm und sich das Produkt bräunlich färbte.

OH-Zahl: 3 mg KOH/g
SZ: 2 mg KOH/g
$T_m$: 99°C, $T_g$: -31°C (DSC, Anlieferungszustand)
Enzym-Test mit Rhizopus arrhizus: ΔDOC: 421 mg/l; zum Vergleich mit PCL: ΔDOC: 2588 mg/l.

## Patentansprüche

**1.** Biologisch abbaubare Polyester P1, erhältlich durch Reaktion einer Mischung, bestehend im wesentlichen aus

(a1) einer Mischung, bestehend im wesentlichen aus

45 bis 80 mol-% Adipinsäure oder esterbildende Derivate davon oder Mischungen davon,

20 bis 55 mol-% Terephthalsäure oder esterbildende Derivate davon oder Mischungen davon, und

0 bis 5 mol-% einer sulfonatgruppenhaltigen Verbindung,

wobei die Summe der einzelnen Molprozentangaben 100 mol-% beträgt, und

(a2) einer Dihydroxyverbindung, ausgewählt aus der Gruppe bestehend aus $C_2$-$C_6$-Alkandiolen und $C_5$-$C_{10}$-Cycloalkandiolen,

wobei man das Molverhältnis von (a1) zu (a2) im Bereich von 0,4:1 bis 1,5:1 wählt, mit der Maßgabe, daß die Polyester P1 ein Molekulargewicht ($M_n$) im Bereich von 5000 bis 50000 g/ mol, eine Viskositätszahl im Bereich von 30 bis 350 g/ml (gemessen in o-Dichlorbenzol/Phenol (Gew.-Verhältnis 50/50) bei einer Konzentration von 0,5 Gew.-% Polyester P1 bei einer Temperatur von 25°C) und einen Schmelzpunkt im Bereich von 50 bis 170°C aufweisen, und mit der weiteren Maßgabe, daß man von 0,01 bis 5 mol-%, bezogen auf die Molmenge der eingesetzten Komponente (a1), eine Verbindung D mit mindestens drei zur Esterbildung befähigten Gruppen zur Herstellung der Polyester P1 einsetzt, sowie der weiteren Maßgabe, daß die Polyester P1 sowohl Hydroxyl- als auch Carboxylendgruppen besitzen, wobei man das Molverhältnis von Carboxylendgruppen zu Hydroxylendgruppen größer als eins wählt.

**2.** Biologisch abbaubare Polyester P2, erhältlich durch Reaktion einer Mischung, bestehend im wesentlichen aus

(b1) einer Mischung, bestehend im wesentlichen aus

25 bis 80 mol-% Adipinsäure oder esterbildende Derivate davon oder Mischungen davon,

20 bis 75 mol-% Terephthalsäure oder esterbildende Derivate davon oder Mischungen davon, und

0 bis 5 mol-% einer sulfonatgruppenhaltigen Verbindung,

wobei die Summe der einzelnen Molprozentangaben 100 mol-% beträgt,

(b2) Dihydroxyverbindung (a2),

wobei man das Molverhältnis von (b1) zu (b2) im Bereich von 0,4:1 bis 1,5:1 wählt,

(b3) von 0,01 bis 100 Gew.-%, bezogen auf Komponente (b1), einer Hydroxycarbonsäure B1, und

(b4) von 0 bis 5 mol-%, bezogen auf Komponente (b1), Verbindung D,

wobei die Hydroxycarbonsäure B1 definiert ist durch die Formeln Ia oder Ib

$$HO-[-C(O)-G-O-]_pH$$

Ia

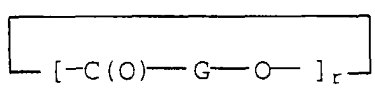

Ib

in deren p eine ganze Zahl von 1 bis 1500 und r eine ganze Zahl von 1 bis 4 bedeuten, und G für einen Rest steht, der ausgewählt ist aus der Gruppe bestehend aus Phenylen, $-(CH_2)_n-$, wobei n eine ganze Zahl von 1 bis 5 bedeutet, -C(R)H- und $-C(R)HCH_2$, wobei R für Methyl oder Ethyl steht,
wobei die Polyester P2 ein Molekulargewicht ($M_n$) im Bereich von 5000 bis 80000 g/mol, eine Viskositätszahl im Bereich von 30 bis 450 g/ml (gemessen in o-Dichlorbenzol/Phenol (Gew.-Verhältnis 50/50) bei einer Konzentration von 0,5 Gew.-% Polyester P2 bei einer Temperatur von 25°C) und einen Schmelzpunkt im Bereich von 50 bis 235°C aufweisen, und sowohl Hydroxyl- als auch Carboxylendgruppen besitzen, wobei man das Molverhältnis von Carboxylendgruppen zu Hydroxylendgruppen größer als eins wählt.

**3.** Biologisch abbaubare Polyester Q1, erhältlich durch Reaktion einer Mischung bestehend im wesentlichen aus

(c1) Polyester P1 und/oder einem Polyester PWD,

(c2) 0,01 bis 50 Gew.-%, bezogen auf (c1), Hydroxycarbonsäure B1, und

(c3) 0 bis 5 mol-%, bezogen auf Komponente (a1) aus der Herstellung von P1 und/oder PWD, Verbindung D, wobei der Polyester PWD erhältlich ist durch Reaktion von im wesentlichen den Komponenten (a1) und (a2), wobei man das Molverhältnis von (a1) zu (a2) im Bereich von 0,4:1 bis 1,5:1 wählt, mit der Maßgabe, daß die Polyester PWD ein Molekulargewicht ($M_n$) im Bereich von 5000 bis 50000 g/mol, eine Viskositätszahl im Bereich von 30 bis 350 g/ml (gemessen in o-Dichlorbenzol/Phenol (Gew.- Verhältnis 50/50) bei einer Konzentration von 0,5 Gew.-% Polyester PWD bei einer Temperatur von 25°C) und einen Schmelzpunkt im Bereich von 50 bis 170°C aufweisen, wobei die Polyester Q1 ein Molekulargewicht ($M_n$) im Bereich von 5000 bis 100000 g/mol, eine Viskositätszahl im Bereich von 30 bis 450 g/ml (gemessen in o-Dichlorbenzol/Phenol (50/50 Gew.-Verhältnis) bei einer Konzentration von 0,5 Gew.-% Polyester Q1 bei einer Temperatur von 25°C) und einen Schmelzpunkt im Bereich von 50 bis 235°C aufweisen, und Polyester PWD als auch Polyester Q1 sowohl Hydroxyl- als auch Carboxylendgruppen besitzen, wobei man das Molverhältnis von Carboxylendgruppen zu Hydroxylendgruppen größer als eins wählt.

**4.** Biologisch abbaubare Polyester Q2 mit einem Molekulargewicht ($M_n$) im Bereich von 6000 bis 60000 g/mol, einer Viskositätszahl im Bereich von 30 bis 340 g/ml (gemessen in o-Dichlorbenzol/Phenol (50/50 Gew.-%) bei einer Konzentration von 0,5 Gew.-% Polyester Q2 bei einer Temperatur von 25°C) und einem Schmelzpunkt im Bereich von 50 bis 170°C, erhältlich durch Reaktion einer Mischung bestehend im wesentlichen aus

(d1) von 95 bis 99,9 Gew.-% Polyester P1 und/oder Polyester PWD gemäß Anspruch 3,

(d2) von 0,1 bis 5 Gew.-% eines Bisoxazolins C1, wobei die Summe der einzelnen Molprozentangaben 100 mol-% beträgt,

(d3) von 0 bis 5 mol-%, bezogen auf Komponente (a1) aus der Herstellung von P1 und/oder PWD, Verbindung D.

**5.** Biologisch abbaubare Polymere T1 mit einem Molekulargewicht ($M_n$) im Bereich von 10 000 bis 100 000 g/mol, mit einer Viskositätszahl im Bereich von 30 bis 450 g/ml (gemessen in o-Dichlorbenzol/Phenol (Gew.-Verhältnis 50/50) bei einer Konzentration von 0,5 Gew.-% Polymer T1 bei einer Temperatur von 25°C) und einem Schmelzpunkt im Bereich von 50 bis 235°C, erhältlich durch Umsetzung des Polyesters Q1 gemäß Anspruch 3 mit (e1) 0,1 bis 5 Gew.-%, bezogen auf den Polyester Q1, Bisoxazolin C1 sowie mit (e2) 0 bis 5 mol-%, bezogen auf Komponente (a1) aus der Herstellung von Polyester Q1 über den Polyester P1 und/oder PWD, Verbindung D.

**6.** Biologisch abbaubare Polymere T2 mit einem Molekulargewicht ($M_n$) im Bereich von 10.000 bis 100.000 g/mol, mit einer Viskositätszahl im Bereich von 30 bis 450 g/ml (gemessen in o-Dichlorbenzol/Phenol (Gew.-Verhältnis 50/50) bei einer Konzentration von 0,5 Gew.-% Polymer T2 bei einer Temperatur von 25°C) und einem Schmelzpunkt im Bereich von 50 bis 235°C, erhältlich durch Umsetzung des Polyesters Q2 mit

(f1) 0,01 bis 50 Gew.-%, bezogen auf Polyester Q2, Hydroxycarbonsäure B1 sowie mit

(f2) 0 bis 5 mol-%, bezogen auf Komponente (a1) aus der Herstellung von Polyester Q2 über den Polyester P1 und/oder PWD, Verbindung D.

**7.** Biologisch abbaubare Polymere T3 mit einem Molekulargewicht ($M_n$) im Bereich von 10.000 bis 100.000 g/mol, mit einer Viskositätszahl im Bereich von 30 bis 450 g/ml (gemessen in o-Dichlorbenzol/Phenol (Gew.-Verhältnis 50/50) bei einer Konzentration von 0,5 Gew.-% Polymer T3 bei einer Temperatur von 25°C) und einem Schmelzpunkt im Bereich von 50 bis 235°C, erhältlich durch Umsetzung von

(g1) Polyester P2, oder

(g2) einer Mischung bestehend im wesentlichen aus Polyester P1 und 0,01 bis 50 Gew.-%, bezogen auf Polyester P1, Hydroxycarbonsäure B1, oder

(g3) einer Mischung bestehend im wesentlichen aus Polyestern P1, die eine unterschiedliche Zusammensetzung voneinander aufweisen,

mit 0,1 bis 5 Gew.-%, bezogen auf die Menge der eingesetzten Polyester, Bisoxazolin C1 sowie mit 0 bis 5 mol-%, bezogen auf die jeweiligen Molmengen an Komponente (a1), die zur Herstellung der eingesetzten Polyester (g1) bis (g3) eingesetzt wurden, Verbindung D.

**8.** Biologisch abbaubare thermoplastische Formmassen T4, erhältlich durch Mischen in an sich bekannter Weise von

(h1) 99,5 bis 0,5 Gew.-% Polyester P1 gemäß Anspruch 1 oder Polyester Q2 gemäß Anspruch 4 oder Polyester PWD gemäß Anspruch 3 mit

(h2) 0,5 bis 99,5 Gew.-% Hydroxycarbonsäure B1.

**9.** Verfahren zur Herstellung der biologisch abbaubaren Polyester P1 gemäß Anspruch 1 in an sich bekannter Weise, dadurch gekennzeichnet, daß man eine Mischung, bestehend im wesentlichen aus
(a1) einer Mischung, bestehend im wesentlichen aus

45 bis 80 mol-% Adipinsäure oder esterbildende Derivate davon oder Mischungen davon,
20 bis 55 mol-% Terephthalsäure oder esterbildende Derivate davon oder Mischungen davon, und
0 bis 5 mol-% einer sulfonatgruppenhaltigen Verbindung,

wobei die Summe der einzelnen Molprozentangaben 100 mol-% beträgt, und
(a2) einer Dihydroxyverbindung, ausgewählt aus der Gruppe bestehend aus $C_2$-$C_6$-Alkandiolen und $C_5$-$C_{10}$-Cycloalkandiolen,
wobei man das Molverhältnis von (a1) zu (a2) im Bereich von 0,4:1 bis 1,5:1 wählt, und von 0,01 bis 5 mol-%, bezogen auf die Molmenge der eingesetzten Komponente (a1), eine Verbindung D mit mindestens drei zur Esterbildung befähigten Gruppen zur Reaktion bringt.

**10.** Verfahren zur Herstellung der biologisch abbaubaren Polyester P2 gemäß Anspruch 2 in an sich bekannter Weise, dadurch gekennzeichnet, daß man eine Mischung, bestehend im wesentlichen aus

(b1) einer Mischung, bestehend im wesentlichen aus

25 bis 80 mol-% Adipinsäure oder esterbildende Derivate davon oder Mischungen davon,

20 bis 75 mol-% Terephthalsäure oder esterbildende Derivate davon oder Mischungen davon, und

0 bis 5 mol-% einer sulfonatgruppenhaltigen Verbindung,

wobei die Summe der einzelnen Molprozentangaben 100 mol-% beträgt,

(b2) Dihydroxyverbindung (a2),

wobei man das Molverhältnis von (b1) zu (b2) im Bereich von 0,4:1 bis 1,5:1 wählt,

(b3) von 0,01 bis 100 Gew.-%, bezogen auf Komponente (b1), einer Hydroxycarbonsäure B1, und

(b4) von 0 bis 5 mol-%, bezogen auf Komponente (b1), Verbindung D,

wobei die Hydroxycarbonsäure B1 definiert ist durch die Formeln Ia oder Ib

$$HO-[-C(O)-G-O-]_pH$$

Ia

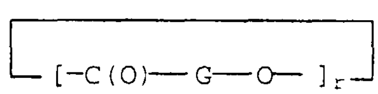

Ib

in der p eine ganze Zahl von 1 bis 1500 und r eine ganze Zahl von 1 bis 4 bedeuten, und G für einen Rest steht, der ausgewählt ist aus der Gruppe bestehend aus Phenylen, $-(CH_2)_n-$, wobei n eine ganze Zahl von 1 bis 5 bedeutet, -C(R)H- und $C(R)HCH_2$, wobei R für Methyl oder Ethyl steht, zur Reaktion bringt.

**11.** Verfahren zur Herstellung der biologisch abbaubaren Polyester Q1 gemäß Anspruch 3 in an sich bekannter Weise, dadurch gekennzeichnet, daß man eine Mischung, bestehend im wesentlichen aus

(c1) Polyester P1 und/oder einem Polyester PWD,

(c2) 0,01 bis 50 Gew.-%, bezogen auf (c1), Hydroxycarbonsäure B1, und

(c3) 0 bis 5 mol-%, bezogen auf Komponente (a1) aus der Herstellung von P1 und/oder PWD, Verbindung D,

zur Reaktion bringt.

**12.** Verfahren zur Herstellung der biologisch abbaubaren Polyester Q2 gemäß Anspruch 4 in an sich bekannter Weise, dadurch gekennzeichnet, daß man eine Mischung, bestehend im wesentlichen aus

(d1) von 95 bis 99,9 Gew.-% Polyester P1 und/oder Polyester PWD gemäß Anspruch 3,

(d2) von 0,1 bis 5 Gew.-% eines Bisoxazolins C1 und

(d3) von 0 bis 5 mol-%, bezogen auf Komponente (a1) aus der Herstellung von P1 und/oder PWD, Verbindung D

zur Reaktion bringt.

**13.** Verfahren zur Herstellung der biologisch abbaubaren Polymeren T1 gemäß Anspruch 5 in an sich bekannter Weise, dadurch gekennzeichnet, daß man Polyester Q1 gemäß Anspruch 3 mit (e1) 0,1 bis 5 Gew.-%, bezogen auf den Polyester Q1, Bisoxazolin C1 sowie mit (e2) 0 bis 5 mol-%, bezogen auf Komponente (a1) aus der Herstellung von Polyester Q1 über Polyester P1 und/oder PWD, Verbindung D zur Reaktion bringt.

**14.** Verfahren zur Herstellung der biologisch abbaubaren Polymeren T2 gemäß Anspruch 6 in an sich bekannter Weise, dadurch gekennzeichnet, daß man Polyester Q2 mit

(f1) 0,01 bis 50 Gew.-%, bezogen auf Polyester Q2, Hydroxycarbonsäure B1 sowie mit

(f2) 0 bis 5 mol-%, bezogen auf Komponente (a1) aus der Herstellung von Polyester Q2 über Polyester P1 und/oder PWD, Verbindung D,

zur Reaktion bringt.

**15.** Verfahren zur Herstellung der biologisch abbaubaren Polymeren T3 gemäß Anspruch 7 in an sich bekannter Weise, dadurch gekennzeichnet, daß man

(g1) Polyester P2, oder

(g2) eine Mischung, bestehend im wesentlichen aus Polyester P1 und 0,01 bis 50 Gew.-%, bezogen auf Polyester P1, Hydroxycarbonsäure B1, oder

(g3) eine Mischung, bestehend im wesentlichen aus Polyestern P1, die eine unterschiedliche Zusammensetzung voneinander aufweisen,

mit 0,1 bis 5 Gew.-%, bezogen auf die Menge der eingesetzten Polyester, Bisoxazolin C1
mit 0 bis 5 mol-%, bezogen auf die jeweiligen Molmengen an Komponente (a1), die zur Herstellung der eingesetzten Polyester (g1) bis (g3) eingesetzt wurden, Verbindung D, zur Reaktion bringt.

**16.** Verfahren zur Herstellung der biologisch abbaubaren thermoplastischen Formmassen T4 gemäß Anspruch 8 in an sich bekannter Weise, dadurch gekennzeichnet, daß man 99,5 bis 0,5 Gew.-% Polyester P1 gemäß Anspruch 1 oder Polyester Q2 gemäß Anspruch 4 oder Polyester PWD gemäß Anspruch 3 mit 0,5 bis 99,5 Gew.-% Hydroxycarbonsäure B1 mischt.

**17.** Verwendung der biologisch abbaubaren Polymere gemäß den Ansprüchen 1 bis 7 oder der thermoplastischen Formmassen gemäß Anspruch 8 oder hergestellt gemäß den Ansprüchen 9 bis 16 zur Herstellung von kompostierbaren Formkörpern.

**18.** Verwendung der biologisch abbaubaren Polymere gemäß den Ansprüchen 1 bis 7 der der thermoplastischen Formmassen gemäß Anspruch 8 oder hergestellt gemäß den Ansprüchen 9 bis 16 zur Herstellung von Klebstoffen.

**19.** Kompostierbare Formkörper, erhältlich durch die Verwendung gemäß Anspruch 17.

**20.** Klebstoffe, erhältlich durch die Verwendung gemäß Anspruch 18.

**21.** Verwendung der biologisch abbaubaren Polymere gemäß den Ansprüchen 1 bis 7 oder der thermoplastischen Formmassen gemäß Anspruch 8 oder hergestellt gemäß den Ansprüchen 9 bis 16 zur Herstellung von biologisch abbaubaren Blends, enthaltend im wesentlichen die erfindungsgemäßen Polymere und Stärke.

**22.** Biologisch abbaubare Blends, erhältlich durch die Verwendung gemäß Anspruch 21.

**23.** Verfahren zur Herstellung biologisch abbaubarer Blends gemäß Anspruch 22 in an sich bekannter Weise, dadurch gekennzeichnet, daß man Stärke mit den erfindungsgemäßen Polymeren mischt.

**24.** Verwendung der biologisch abbaubaren Polymere gemäß den Ansprüchen 1 bis 7 oder der thermoplastischen Formmassen gemäß Anspruch 8 oder hergestellt gemäß den Ansprüchen 9 bis 16 zur Herstellung von biologisch abbaubaren Schäumen.

**25.** Biologisch abbaubare Schäume, erhältlich durch die Verwendung gemäß Anspruch 24.

**26.** Verwendung der biologisch abbaubaren Polymere gemäß den Ansprüchen 1 bis 7 oder der thermoplastischen Formmassen gemäß Anspruch 8 oder hergestellt gemäß den Ansprüchen 9 bis 16 zur Herstellung von Papierbeschichtungsmitteln.

**27.** Papierbeschichtungsmittel, erhältlich durch die Verwendung gemäß Anspruch 26.

**28.** Biologisch abbaubare Polyester Q2 nach Anspruch 4, worin als Komponente (d2) ein Bis(2-oxazolinyl)benzol verwendet wird.

**29.** Biologisch abbaubare Polyester Q2 nach Anspruch 28, worin als Komponente (d2) 1,3-Bis(2-oxazolinyl)benzol verwendet wird.

## Claims

**1.** Biodegradable polyesters P1 obtainable by reaction of a mixture consisting essentially of

(a1) a mixture consisting essentially of

from 45 to 80 mol% of adipic acid or ester-forming derivatives thereof or mixtures thereof,

from 20 to 55 mol% of terephthalic acid or ester-forming derivatives thereof or mixtures thereof, and

from 0 to 5 mol% of a sulfonate compound,

the sum of the individual mole percentages being 100 mol%, and

(a2) a dihydroxy compound selected from the group consisting of $C_2$-$C_6$-alkanediols and $C_5$-$C_{10}$-cycloalkanediols,

the molar ratio of (a1) to (a2) being chosen within the range from 0.4:1 to 1.5:1, with the proviso that the polyesters P1 have a molecular weight ($M_n$) within the range from 5000 to 50,000 g/mol, a viscosity number within the range from 30 to 350 g/ml (measured in 50:50 w/w o-dichlorobenzene/phenol at a concentration of 0.5% by weight of polyester P1 at 25°C) and a melting point within the range from 50 to 170°C and with the further proviso that the polyesters P1 are prepared using from 0.01 to 5 mol%, based on the molar quantity used of component (a1), of a compound D having at least three groups capable of ester formation, and the further proviso that the polyesters P1 have both hydroxyl and carboxyl end groups, the molar ratio of carboxyl end groups to hydroxyl end groups being chosen to be greater than one.

**2.** Biodegradable polyesters P2 obtainable by reaction of a mixture consisting essentially of

(b1) a mixture consisting essentially of

from 25 to 80 mol% of adipic acid or ester-forming derivatives thereof or mixtures thereof,

from 20 to 75 mol% of terephthalic acid or ester-forming derivatives thereof or mixtures thereof, and

from 0 to 5 mol% of a sulfonate compound,

the sum of the individual mole percentages being 100 mol%,

(b2) dihydroxy compound (a2),

the molar ratio of (b1) to (b2) being chosen within the range from 0.4:1 to 1.5:1,

(b3) from 0.01 to 100% by weight, based on component (b1), of a hydroxycarboxylic acid B1, and

(b4) from 0 to 5 mol%, based on component (b1), of compound D,

the hydroxycarboxylic acid B1 being defined by the formula Ia or Ib

$$HO-[-C(O)-G-O-]_pH$$

Ia

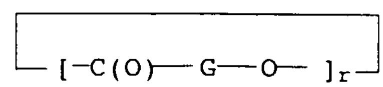

Ib

where p is an integer from 1 to 1500, r is an integer from 1 to 4, and G is a radical selected from the group consisting of phenylene, $-(CH_2)_n-$, where n is an integer from 1 to 5, -C(R)H- and $-C(R)HCH_2$, where R is methyl or ethyl, the polyesters P2 having a molecular weight ($M_n$) within the range from 5000 to 80,000 g/mol, a viscosity number within the range from 30 to 450 g/ml (measured in 50:50 w/w o-dichlorobenzene/phenol at a concentration of 0.5% by weight of polyester P2 at 25°C) and a melting point within the range from 50 to 235°C, and having both hydroxyl and carboxyl end groups, the molar ratio of carboxyl end groups to hydroxyl end groups being chosen to be greater than one.

**3.** Biodegradable polyesters Q1 obtainable by reaction of a mixture consisting essentially of

(c1) polyester P1 and/or a polyester PWD,

(c2) from 0.01 to 50% by weight, based on (c1), of hydroxycarboxylic acid B1, and

(c3) from 0 to 5 mol%, based on component (a1) from the preparation of P1 and/or PWD, of compound D, the polyester PWD being obtainable by reaction of essentially components (a1) and (a2), the molar ratio of (a1) to (a2) being chosen within the range from 0.4:1 to 1.5:1, with the proviso that the polyesters PWD have a molecular weight ($M_n$) within the range from 5000 to 50,000 g/mol, a viscosity number within the range from 30 to 350 g/ml (measured in 50:50 w/w o-dichlorobenzene/phenol at a concentration of 0.5% by weight of polyester PWD at 25°C) and a melting point within the range from 50 to 170°C, the polyesters Q1 having a

molecular weight ($M_n$) within the range from 5000 to 100,000 g/mol, a viscosity number within the range from 30 to 450 g/ml (measured in 50:50 w/w o-dichlorobenzene/phenol at a concentration of 0.5% by weight of polyester Q1 at 25°C) and a melting point within the range from 50 to 235°C, and polyester PWD and polyester Q1 having both hydroxyl and carboxyl end groups, the molar ratio of carboxyl end groups to hydroxyl end groups being chosen to be greater than one.

**4.** Biodegradable polyesters Q2 having a molecular weight ($M_n$) within the range from 6000 to 60,000 g/mol, a viscosity number within the range from 30 to 340 g/ml (measured in 50:50% by weight o-dichlorobenzene/phenol at a concentration of 0.5% by weight of polyester Q2 at 25°C) and a melting point within the range from 50 to 170°C and obtainable by reaction of a mixture consisting essentially of

(d1) from 95 to 99.9% by weight of polyester P1 and/or polyester PWD as set forth in claim 3,

(d2) from 0.1 to 5% by weight of a bisoxazoline C1, where the sum of the individual mole percentages is 100 mol%,

(d3) from 0 to 5 mol%, based on component (a1) from the preparation of P1 and/or PWD, of compound D.

**5.** Biodegradable polymers T1 having a molecular weight ($M_n$) within the range from 10,000 to 100,000 g/mol, a viscosity number within the range from 30 to 450 g/ml (measured in 50:50 w/w o-dichlorobenzene/phenol at a concentration of 0.5% by weight of polymer T1 at 25°C) and a melting point within the range from 50 to 235°C and obtainable by reaction of the polyester Q1 as set forth in claim 3 with (e1) from 0.1 to 5% by weight, based on the polyester Q1, of bisoxazoline C1 and with (e2) from 0 to 5 mol%, based on component (a1) from the preparation of polyester Q1 via polyester P1 and/or PWD, of compound .D.

**6.** Biodegradable polymers T2 having a molecular weight ($M_n$) within the range from 10,000 to 100,000 g/mol, a viscosity number within the range from 30 to 450 g/ml (measured in 50:50 w/w o-dichlorobenzene/phenol at a concentration of 0.5% by weight of polymer T2 at 25°C) and a melting point within the range from 50 to 235°C and obtainable by reaction of the polyester Q2 with

(f1) from 0.01 to 50% by weight, based on polyester Q2, of hydroxycarboxylic acid B1 and also with

(f2) from 0 to 5 mol%, based on component (a1) from the preparation of polyester Q2 via polyester P1 and/or PWD, of compound D.

**7.** Biodegradable polymers T3 having a molecular weight ($M_n$) within the range from 10,000 to 100,000 g/mol, a viscosity number within the range from 30 to 450 g/ml (measured in 50:50 w/w o-dichlorobenzene/phenol at a concentration of 0.5% by weight of polymer T3 at 25°C) and a melting point within the range from 50 to 235°C and obtainable by reaction of

(g1) polyester P2, or

(g2) a mixture consisting essentially of polyester P1 and from 0.01 to 50% by weight, based on polyester P1, of hydroxycarboxylic acid B1, or

(g3) a mixture consisting essentially of polyesters P1 which have mutually different compositions,

with from 0.1 to 5% by weight, based on the amount of polyesters used, of bisoxazoline C1 and also
with from 0 to 5 mol%, based on the respective molar quantities of component (a1) used for preparing the polyesters (g1) to (g3) used, of compound D.

**8.** Biodegradable thermoplastic molding compositions T4 obtainable by conventional mixing of

(h1) from 99.5 to 0.5% by weight of polyester P1 as set forth in claim 1 or polyester Q2 as set forth in claim 4 or polyester PWD as set forth in claim 3 with

(h2) from 0.5 to 99.5% by weight of hydroxycarboxylic acid B1.

**9.** A process for preparing the biodegradable polyesters P1 of claim 1 in a conventional manner, which comprises reacting a mixture consisting essentially of

(a1) a mixture consisting essentially of

from 45 to 80 mol% of adipic acid or ester-forming derivatives thereof or mixtures thereof,
from 20 to 55 mol% of terephthalic acid or ester-forming derivatives thereof or mixtures thereof, and
from 0 to 5 mol% of a sulfonate compound,

the sum of the individual mole percentages being 100 mol%, and

(a2) a dihydroxy compound selected from the group consisting of $C_2$-$C_6$-alkanediols and $C_5$-$C_{10}$-cycloalkanediols,

the molar ratio of (a1) to (a2) being chosen within the range from 0.4:1 to 1.5:1, and from 0.01 to 5 mol%, based on the molar quantity used of component (a1), of a compound D having at least three groups capable of ester formation.

**10.** A process for preparing the biodegradable polyesters P2 of claim 2 in a conventional manner, which comprises reacting a mixture consisting essentially of

(b1) a mixture consisting essentially of

from 25 to 80 mol% of adipic acid or ester-forming derivatives thereof or mixtures thereof,

from 20 to 75 mol% of terephthalic acid or ester-forming derivatives thereof or mixtures thereof, and

from 0 to 5 mol% of a sulfonate compound,

the sum of the individual mole percentages being 100 mol%,

(b2) dihydroxy compound (a2),

the molar ratio of (b1) to (b2) being chosen within the range from 0.4:1 to 1.5:1,

(b3) from 0.01 to 100% by weight, based on component (b1), of a hydroxycarboxylic acid B1, and

(b4) from 0 to 5 mol%, based on component (b1), of compound D,

the hydroxycarboxylic acid B1 being defined by the formula Ia or Ib

$$HO-[-C(O)-G-O-]_pH$$

Ia

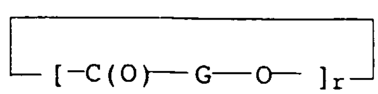

Ib

where p is an integer from 1 to 1500, r is an integer from 1 to 4, and G is a radical selected from the group consisting of phenylene, $-(CH_2)_n-$, where n is an integer from 1 to 5, -C(R)H- and $-C(R)HCH_2$, where R is methyl or ethyl.

**11.** A process for preparing the biodegradable polyesters Q1 of claim 3 in a conventional manner, which comprises reacting a mixture consisting essentially of

(c1) polyester P1 and/or a polyester PWD,

(c2) from 0.01 to 50% by weight, based on (c1), of hydroxycarboxylic acid B1, and

(c3) from 0 to 5 mol%, based on component (a1) from the preparation of P1 and/or PWD, of compound D.

**12.** A process for preparing the biodegradable polyesters Q2 of claim 4 in a conventional manner, which comprises reacting a mixture consisting essentially of

(d1) from 95 to 99.9% by weight of polyester P1 and/or polyester PWD as set forth in claim 3,

(d2) from 0.1 to 5% by weight of a bisoxazoline C1, and

(d3) from 0 to 5 mol%, based on component (a1) from the preparation of P1 and/or PWD, of compound D.

**13.** A process for preparing the biodegradable polymers T1 of claim 5 in a conventional manner, which comprises reacting polyester Q1 as set forth in claim 3 with (e1) from 0.1 to 5% by weight, based on the polyester Q1, of bisoxazoline C1 and also with (e2) from 0 to 5 mol%, based on component (a1) from the preparation of polyester Q1 via polyester P1 and/or PWD, of compound D.

**14.** A process for preparing the biodegradable polymers T2 of claim 6 in a conventional manner, which comprises reacting polyester Q2 with

(f1) from 0.01 to 50% by weight, based on polyester Q2, of hydroxycarboxylic acid B1 and also with

(f2) from 0 to 5 mol%, based on component (a1) from the preparation of polyester Q2 via polyester P1 and/or PWD, of compound D.

**15.** A process for preparing the biodegradable polymers T3 of claim 7 in a conventional manner, which comprises reacting

(g1) polyester P2, or

(g2) a mixture consisting essentially of polyester P1 and from 0.01 to 50% by weight, based on polyester P1, of hydroxycarboxylic acid B1, or

(g3) a mixture consisting essentially of polyesters P1 which have mutually different compositions,

with from 0.1 to 5% by weight, based on the amount of polyesters used, of bisoxazoline C1
with from 0 to 5 mol%, based on the respective molar quantities of component (a1) used for preparing the polyesters (g1) to (g3) used, of compound D.

**16.** A process for preparing the biodegradable thermoplastic molding compositions T4 of claim 8 in a conventional manner, which comprises mixing from 99.5 to 0.5% by weight of polyester P1 as set forth in claim 1 or polyester Q2 as set forth in claim 4 or polyester PWD as set forth in claim 3 with from 0.5 to 99.5% by weight of hydroxycarboxylic acid B1.

**17.** The use of the biodegradable polymers of claims 1 to 7 or of the thermoplastic molding compositions of claim 8 or prepared as claimed in claims 9 to 16 for producing compostable moldings.

**18.** The use of the biodegradable polymers of claims 1 to 7 or of the thermoplastic molding compositions of claim 8 or prepared as claimed in claims 9 to 16 for producing adhesives.

**19.** Compostable moldings obtainable by the use claimed in claim 17.

**20.** Adhesives obtainable by the use claimed in claim 18.

**21.** The use of the biodegradable polymers of claims 1 to 7 or of the thermoplastic molding compositions of claim 8 or prepared as claimed in claims 9 to 16 for producing biodegradable blends containing essentially the polymers of the invention and starch.

**22.** Biodegradable blends obtainable by the use claimed in claim 21.

**23.** A process for producing biodegradable blends as claimed in claim 22 in a conventional manner, which comprises mixing starch with the polymers of the invention.

**24.** The use of the biodegradable polymers of claims 1 to 7 or of the thermoplastic molding compositions of claim 8 or prepared as claimed in claims 9 to 16 for producing biodegradable foams.

**25.** Biodegradable foams obtainable by the use claimed in claim 24.

**26.** The use of the biodegradable polymers of claims 1 to 7 or of the thermoplastic molding compositions of claim 8 or prepared as claimed in claims 9 to 16 for producing paper coating compositions.

**27.** Paper coating compositions obtainable by the use claimed in claim 26.

**28.** Biodegradable polyesters Q2 as claimed in claim 4, in which a bis(2-oxazolinyl)benzene is used as component (d2).

**29.** Biodegradable polyesters Q2 as claimed in claim 28, in which a 1,3-bis(2-oxazolinyl)benzene is used as component (d2).

**Revendications**

**1.** Polyesters biologiquement dégradables P1, que l'on peut obtenir par réaction d'un mélange, constitué essentiellement

(a1) d'un mélange, constitué essentiellement

de 45 à 80 moles % d'acide adipique ou de dérivés estérogènes de celui-ci ou de leurs mélanges,
de 20 à 55 moles % d'acide téréphtalique ou de dérivés estérogènes de celui-ci ou de leurs mélanges, et
de 0 à 5 moles % d'un composé contenant des groupes sulfonate,
la somme des différentes indications en moles % étant de 100 moles %, et

(a2) d'un composé dihydroxy, choisi parmi le groupe comprenant des alcanediols en $C_2$-$C_6$ et des cycloalcanediols en $C_5$-$C_{10}$,

le rapport molaire entre (a1) et (a2) étant choisi dans la gamme de 0,4/1 à 1,5/1, à la condition que les polyesters P1 présentent un poids moléculaire ($M_n$) de l'ordre de 5.000 à 50.000 g/mole, un indice de viscosité de l'ordre de 30 à 350 g/ml [mesuré dans du o-dichlorobenzène/phénol (rapport pondéral de 50/50) à une concentration de 0,5% en poids de polyesters P1 à une température de 25°C] et un point de fusion de l'ordre de 50 à 170°C, et à la condition supplémentaire qu'on met en oeuvre de 0,01 à 5 moles %, par rapport à la quantité molaire du composant (a1) mis en oeuvre, d'un composé D présentant au moins trois groupes capables de former des esters pour la préparation des polyesters P1, ainsi qu'à la condition supplémentaire que les polyesters P1 possèdent des groupes terminaux aussi bien hydroxyle que carboxyle, le rapport molaire entre les groupes terminaux carboxyle et les groupes terminaux hydroxyle étant choisi plus grand que un.

**2.** Polyesters biologiquement dégradables P2 que l'on peut obtenir par réaction d'un mélange, constitué essentiellement

(b1) d'un mélange, constitué essentiellement

de 25 à 80 moles % d'acide adipique ou de dérivés estérogènes de celui-ci ou de leurs mélanges,
de 20 à 75 moles % d'acide téréphtalique ou de dérivés estérogènes de celui-ci ou de leurs mélanges, et
de 0 à 5 moles % d'un composé contenant des groupes sulfonate,
la somme des différentes indications en moles % étant de 100 moles %, et

(b2) d'un composé dihydroxy (a2),

le rapport molaire entre (b1) et (b2) étant choisi dans la gamme de 0,4/1 à 1,5/1,

(b3) de 0,01 à 100% en poids, par rapport au composant (b1), d'un acide hydroxycarboxylique B1, et
(b4) de 0 à 5 moles %, par rapport au composant (b1), de composé D,

l'acide hydroxycarboxylique B1 étant défini par les formules Ia ou Ib :

$$HO-[-C(O)-G-O-]_pH$$

**Ia**

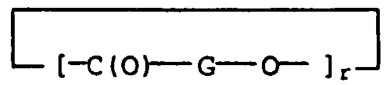

**Ib**

dans lesquelles p est un nombre entier de 1 à 1.500 et r un nombre entier de 1 à 4, et G représente un radical qui est choisi parmi le groupe comprenant du phénylène, $-(CH_2)_n-$, où n est un nombre entier de 1 à 5, -C(R)H- et $-C(R)HCH_2$, où R représente du méthyle ou de l'éthyle,

les polyesters P2 présentant un poids moléculaire ($M_n$) de l'ordre de 5.000 à 80.000 g/mole, un indice de viscosité de l'ordre de 30 à 450 g/ml [mesuré dans du o-dichlorobenzène/phénol (rapport pondéral de 50/50) à une concentration de 0,5% en poids de polyesters P2 à une température de 25°C] et un point de fusion de l'ordre de 50 à 235°C, et possédant des groupes terminaux aussi bien hydroxyle que carboxyle, le rapport molaire entre les groupes terminaux carboxyle et les groupes terminaux hydroxyle étant choisis plus grand que un.

**3.** Polyesters biologiquement dégradables Q1, que l'on peut obtenir par réaction d'un mélange constitué essentiellement

(c1) de polyesters P1 et/ou d'un polyester PWD,
(c2) de 0,01 à 50% en poids, par rapport à (c1), d'acide hydroxycarboxylique B1, et
(c3) de 0 à 5 moles %, par rapport au composant (a1) de la préparation de P1 et/ou de PWD, de composé D, le polyester PWD pouvant être obtenu par réaction essentiellement des composants (a1) et (a2), le rapport molaire entre (a1) et (a2) étant choisi dans la gamme de 0,4/1 à 1,5/1, à la condition que les polyesters PWD présentent un poids moléculaire ($M_n$) de l'ordre de 5.000 à 50.000 g/mole, un indice de viscosité de l'ordre de 30 à 350 g/ml [mesuré dans du o-dichlorobenzène/phénol (rapport pondéral de 50/50) à une concentration de 0,5% en poids de polyesters PWD à une température de 25°C] et un point de fusion de l'ordre de 50 à 170°C, les polyesters Q1 présentant un poids moléculaire ($M_n$) de l'ordre de 5.000 à 100.000 g/mole, un indice de viscosité de l'ordre de 30 à 450 g/ml [mesuré dans du o-dichlorobenzène/phénol (rapport pondéral de 50/50) à une concentration de 0,5% en poids de polyesters Q1 à une température de 25°C] et un point de fusion de l'ordre de 50 à 235°C, les polyesters PWD possédant de même que les polyesters Q1 des groupes terminaux aussi bien hydroxyle que carboxyle, le rapport molaire entre les groupes terminaux carboxyle et hydroxyle étant choisi plus grand que un.

**4.** Polyesters biologiquement dégradables Q2, présentant un poids moléculaire ($M_n$) de l'ordre de 6.000 à 60.000 g/mole, un indice de viscosité de l'ordre de 30 à 340 g/ml [mesuré dans du o-dichlorobenzène/phénol (50/50% en poids) à une concentration de 0,5% en poids de polyesters Q2 à une température de 25°C] et un point de fusion de l'ordre de 50 à 170°C, que l'on peut obtenir par réaction d'un mélange constitué essentiellement

(d1) de 95 à 99,9% en poids de polyesters P1 et/ou de polyesters PWD suivant la revendication 3,
(d2) de 0,1 à 5% en poids d'une bisoxazoline C1, la somme des différentes indications en moles % étant de 100 moles %,
(d3) de 0 à 5 moles %, par rapport au composant (a1) de la préparation de P1 et/ou de PWD, de composé D.

**5.** Polymères biologiquement dégradables T1 présentant un poids moléculaire ($M_n$) de l'ordre de 10.000 à 100.000 g/mole, un indice de viscosité de l'ordre de 30 à 450 g/ml [mesuré dans du o-dichlorobenzène/phénol (rapport pondéral de 50/50) à une concentration de 0,5% en poids de polymères T1 à une température de 25°C] et un point de fusion de l'ordre de 50 à 235°C, que l'on peut obtenir par réaction du polyester Q1 suivant la revendication 3 avec (e1) 0,1 à 5% en poids, par rapport au polyester Q1, de bisoxazoline C1 ainsi qu'avec (e2) 0 à 5 moles %, par rapport au composant (a1) de la préparation de polyester Q1 par l'intermédiaire du polyester P1 et/ou PWD, de composé D.

**6.** Polymères biologiquement dégradables T2 présentant un poids moléculaire ($M_n$) de l'ordre de 10.000 à 100.000

g/mole, un indice de viscosité de l'ordre de 30 à 450 g/ml [mesuré dans du o-dichlorobenzène/phénol (rapport pondéral de 50/50) à une concentration de 0,5% en poids de polymères T2 à une température de 25°C] et un point de fusion de l'ordre de 50 à 235°C, que l'on peut obtenir par réaction du polyester Q2 avec

(f1) 0,01 à 50 % en poids, par rapport au polyester Q2, d'acide hydroxycarboxylique B1, ainsi qu'avec
(f2) 0 à 5 moles %, par rapport au composant (a1) de la préparation de polyester Q2 par l'intermédiaire du polyester P1 et/ou PWD, de composé D.

**7.** Polymères biologiquement dégradables T3 présentant un poids moléculaire ($M_n$) de l'ordre de 10.000 à 100.000 g/mole, un indice de viscosité de l'ordre de 30 à 450 g/ml [mesuré dans du o-dichlorobenzène/phénol (rapport pondéral de 50/50) à une concentration de 0,5% en poids de polymère T3 à une température de 25°C] et un point de fusion de l'ordre de 50 à 235°C, que l'on peut obtenir par réaction

(g1) de polyesters P2, ou
(g2) d'un mélange constitué essentiellement de polyesters P1 et de 0,01 à 50% en poids, par rapport aux polyesters P1, d'acide hydroxycarboxylique B1, ou
(g3) d'un mélange constitué essentiellement de polyesters P1, qui présentent une composition différente l'un de l'autre,

avec 0,1 à 5% en poids, par rapport à la quantité des polyesters mis en oeuvre, de bisoxazoline C1, ainsi que avec 0 à 5 moles %, par rapport à la quantité moléculaire respective de composant (a1) qui a été mise en oeuvre pour la préparation des polyesters introduits (g1) à (g3), de composé D.

**8.** Masses de moulage thermoplastiques biologiquement dégradables T4, que l'on peut obtenir par mélange d'une manière connue en soi

(h1) de 99,5 à 0,5% en poids de polyesters P1 suivant la revendication 1 ou de polyesters Q2 suivant la revendication 4 ou de polyesters PWD suivant la revendication 3, avec
(h2) 0,5 à 99,5% en poids d'acide hydroxycarboxylique B1.

**9.** Procédé de préparation des polyesters biologiquement dégradables P1 suivant la revendication 1 d'une manière connue en soi, caractérisé en ce qu'on fait réagir un mélange constitué essentiellement

(a1) d'un mélange, constitué essentiellement

de 45 à 80 moles % d'acide adipique ou de dérivés estérogènes de celui-ci ou de leurs mélanges,
de 20 à 55 moles % d'acide téréphtalique ou de dérivés estérogènes de celui-ci ou de leurs mélanges, et
de 0 à 5 moles % d'un composé contenant des groupes sulfonate,
la somme des différentes indications en moles % étant de 100 moles %, et

(a2) d'un composé dihydroxy, choisi parmi le groupe comprenant des alcanediols en $C_2$-$C_6$ et des cycloalcanediols en $C_5$-$C_{10}$,

le rapport molaire entre (a1) et (a2) étant choisi dans la gamme de 0,4/1 à 1,5/1, et de 0,01 à 5 moles %, par rapport à la quantité molaire du composant (a1) mis en oeuvre, d'un composé D portant au moins trois groupes capables de former des esters.

**10.** Procédé de préparation des polyesters biologiquement dégradables P2 suivant la revendication 2 d'une manière connue en soi, caractérisé en qu'on fait réagir un mélange, constitué essentiellement

(b1) d'un mélange, constitué essentiellement

de 25 à 80 moles % d'acide adipique ou de dérivés estérogènes de celui-ci ou de leurs mélanges,
de 20 à 75 moles % d'acide téréphtalique ou de dérivés estérogènes de celui-ci ou de leurs mélanges, et
de 0 à 5 moles % d'un composé contenant des groupes sulfonate,
la somme des différentes indications en moles % étant de 100 moles %, et

(b2) d'un composé dihydroxy (a2),

le rapport molaire entre (b1) et (b2) étant choisi dans la gamme de 0,4/1 à 1,5/1,

(b3) de 0,01 à 100% en poids, par rapport au composant (b1), d'un acide hydroxycarboxylique B1, et
(b4) de 0 à 5 moles %, par rapport au composant (b1), de composé D,

l'acide hydroxycarboxylique B1 étant défini par les formules Ia ou Ib :

$$HO-[-C(O)-G-O-]_pH$$

**Ia**

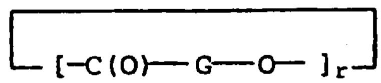

**Ib**

dans lesquelles p est un nombre entier de 1 à 1.500 et r un nombre entier de 1 à 4, et G représente un radical qui est choisi parmi le groupe comprenant du phénylène, $-(CH_2)_n-$, où n est un nombre entier de 1 à 5, -C(R)H- et $-C(R)HCH_2$, où R représente du méthyle ou de l'éthyle.

**11.** Procédé de préparation des polyesters biologiquement dégradables Q1 suivant la revendication 3 d'une manière connue en soi, caractérisé en ce qu'on met à réagir un mélange, constitué essentiellement

(c1) de polyesters P1 et/ou d'un polyester PWD,
(c2) de 0,01 à 50% en poids, par rapport à (c1), d'acide hydroxycarboxylique B1, et
(c3) de 0 à 5 moles %, par rapport au composant (a1) de la préparation de P1 et/ou de PWD, de composé D.

**12.** Procédé de préparation des polyesters biologiquement dégradables Q2 suivant la revendication 4 d'une manière connue en soi, caractérisé en ce qu'on met à réagir un mélange constitué essentiellement

(d1) de 95 à 99,9% en poids de polyesters P1 et/ou de polyesters PWD suivant la revendication 3,
(d2) de 0,1 à 5% en poids d'une bisoxazoline C1, et
(d3) de 0 à 5 moles %, par rapport au composant (a1) de la préparation de P1 et/ou de PWD, de composé D.

**13.** Procédé de préparation des polymères biologiquement dégradables T1 suivant la revendication 5 d'une manière connue en soi, caractérisé en ce qu'on met à réagir des polyesters Q1 suivant la revendication 3 avec (e1) 0,1 à 5% en poids, par rapport aux polyesters Q1, de bisoxazoline C1 ainsi qu'avec (e2) 0 à 5 moles %, par rapport au composant (a1) de la préparation de polyesters Q1 par l'intermédiaire du polyester P1 et/ou PWD, de composé D.

**14.** Procédé de préparation des polymères biologiquement dégradables T2 suivant la revendication 6 d'une manière connue en soi, caractérisé en ce qu'on met à réagir des polyesters Q2 avec

(f1) 0,01 à 50% en poids, par rapport aux polyesters Q2, d'acide hydroxycarboxylique B1, ainsi qu'avec
(f2) 0 à 5 moles %, par rapport au composant (a1) de la préparation de polyesters Q2 par l'intermédiaire de polyesters P1 et/ou PWD, de composé D.

**15.** Procédé de préparation des polymères biologiquement dégradables T3 suivant la revendication 7 d'une manière connue en soi, caractérisé en ce qu'on met à réagir

(g1) du polyester P2, ou
(g2) un mélange constitué essentiellement de polyesters P1 et de 0,01 à 50% en poids, par rapport aux polyesters P1, d'acide hydroxycarboxylique B1, ou
(g3) un mélange constitué essentiellement de polyesters P1, qui présentent une composition différente l'un de l'autre,

avec 0,1 à 5% en poids, par rapport à la quantité des polyesters mis en oeuvre, de bisoxazoline C1,
avec 0 à 5 moles %, par rapport aux quantités molaires respectives du composant (a1), qui a été mis en oeuvre pour la préparation des polyesters mis en oeuvre (g1) à (g3), de composé D.

**16.** Procédé de préparation des masses de moulage thermoplastiques biologiquement dégradables T4 suivant la

revendication 8 d'une manière connue en soi, caractérisé en ce qu'on mélange 99,5 à 0,5% en poids de polyesters P1 suivant la revendication 1 ou de polyesters Q2 suivant la revendication 4 ou de polyesters PWD suivant la revendication 3 avec 0,5 à 99,5% en poids d'acide hydroxycarboxylique B1.

**17.** Utilisation des polymères biologiquement dégradables suivant les revendications 1 à 7 ou des masses de moulage thermoplastiques suivant la revendication 8 ou préparés suivant l'une des revendications 9 à 16, pour la fabrication de corps façonnés compostables.

**18.** Utilisation des polymères biologiquement dégradables suivant l'une des revendications 1 à 7 ou des masses de moulage thermoplastiques suivant la revendication 8 ou préparés suivant l'une des revendications 9 à 16, pour la fabrication d'adhésifs.

**19.** Corps façonnés compostables, que l'on peut obtenir par l'utilisation suivant la revendication 17.

**20.** Adhésif, que l'on peut obtenir par l'utilisation suivant la revendication 18.

**21.** Utilisation des polymères biologiquement dégradables suivant les revendications 1 à 7 ou des masses de moulage thermoplastiques suivant la revendication 8 ou préparés selon l'une des revendications 9 à 16, pour la fabrication de mélanges biologiquement dégradables, contenant essentiellement les polymères suivant l'invention et de l'amidon.

**22.** Mélanges biologiquement dégradables, que l'on peut obtenir par l'utilisation suivant la revendication 21.

**23.** Procédé de préparation de mélanges biologiquement dégradables suivant la revendication 22 d'une manière connue en soi, caractérisé en ce que l'on mélange de l'amidon avec les polymères suivant l'invention.

**24.** Utilisation des polymères biologiquement dégradables suivant l'une des revendications 1 à 7 ou des masses de moulage thermoplastiques suivant la revendication 8 ou préparés suivant l'une des revendications 9 à 16, pour la fabrication de mousses biologiquement dégradables.

**25.** Mousses biologiquement dégradables, que l'on peut obtenir par l'utilisation suivant la revendication 24.

**26.** Utilisation des polymères biologiquement dégradables suivant l'une des revendications 1 à 7 ou des masses de moulage thermoplastique suivant la revendication 8 ou préparés suivant l'une des revendications 9 à 16, pour la fabrication de produits de couchage de papier.

**27.** Produits de couchage de papier, que l'on peut obtenir par l'utilisation suivant la revendication 26.

**28.** Polyesters biologiquement dégradables Q2 suivant la revendication 4, caractérisés en ce qu'on utilise, comme composant (d2), un bis(2-oxazolinyl)benzène.

**29.** Polyesters biologiquement dégradables Q2 suivant la revendication 28, caractérisés en ce qu'on utilise, comme composant (d2), du 1,3-bis(2-oxazolinyl)benzène.